# EUROPEAN PATENT APPLICATION

(11) **EP 3 748 363 A1**
(43) Date of publication of application: **09.12.2020**
(21) Application number: 19746592.5
(22) Date of filing: 09.01.2019
(51) Int. Cl.: G01N 33/68, C12N 15/85, C12N 5/0735, C12Q 1/68

(54) **METHOD FOR HIGH-THROUGHPUT ANALYZING PROTEINS AND APPLICABLE LIBRARY THEREOF**

(30) Priority: 31.01.2018 CN 201810096299
(71) Applicant: Center for Excellence in Molecular Cell Science, Chinese Academy of Sciences, Xuhui District Shanghai 200031 (CN)
(72) Inventor: LI, Jinsong, Shanghai 200031 (CN); JIANG, Jing, Shanghai 200031 (CN); ZHAO, Yun, Shanghai 200031 (CN); LI, Lin, Shanghai 200031 (CN); LI, Dangsheng, Shanghai 200031 (CN)
(74) Representative: Herrero & Asociados, S.L.
(86) International application number: PCT/CN2019/071005
(87) International publication number: WO 2019/149040

(57) **Abstract**

The present disclosure relates to the field of biology, and provides a high-throughput protein analysis method and a suitable library thereof. The high-throughput protein analysis method of the present disclosure includes: using a tagged semi-cloned mouse library to perform parallel indicator analysis on a plurality of different target proteins of interest with one or several tag protein antibodies. In the tagged semi-cloned mouse library, each semi-cloned mouse is a semi-cloned mouse obtained by culturing after injecting an androgenetic haploid embryonic stem cell into an ovum, or a sexually propagated progeny thereof. The androgenetic haploid embryonic stem cell contains a gene that expresses a fusion protein of a target protein of interest and a tag protein, and the semi-cloned mouse can express the fusion protein of the target protein of interest and the tag protein. The present disclosure provides a system suitable for high-throughput in vivo, real-time and dynamic research for research on biomacromolecules.

SEQUENCE LISTING

<110>  Shanghai Institutes for Biological Sciences, Chinese
       Academy of Sciences

<120>  High-Throughput Protein Analysis Method and Suitable Library
       Thereof

<130>  PWWEP200243-MO

<160>  59

<170>  PatentIn version 3.5

<210>  1
<211>  44
<212>  PRT
<213>  Artificial Sequence

<220>
<223>  The amino acid sequence of Brd4-N-ATF label

<400>  1

Gly Leu Asn Asp Ile Phe Glu Ala Gln Lys Ile Glu Trp His Glu Glu
1               5                   10                  15

Asn Leu Tyr Phe Gln Gly Asp Tyr Lys Asp His Asp Gly Asp Tyr Lys
            20                  25                  30

Asp His Asp Ile Asp Tyr Lys Asp Asp Asp Asp Lys
        35                  40

<210>  2
<211>  44
<212>  PRT
<213>  Artificial Sequence

<220>
<223>  The amino acid sequence of Brd4-C-FTA label

<400>  2

Asp Tyr Lys Asp His Asp Gly Asp Tyr Lys Asp His Asp Ile Asp Tyr
1               5                   10                  15

Lys Asp Asp Asp Asp Lys Glu Asn Leu Tyr Phe Gln Gly Gly Leu Asn
            20                  25                  30

Asp Ile Phe Glu Ala Gln Lys Ile Glu Trp His Glu
        35                  40

<210>  3
<211>  31
<212>  PRT
<213>  Artificial Sequence

<220>
<223>  The amino acid sequence of Brd4-C-HTA label

<400>  3

Tyr Pro Tyr Asp Val Pro Asp Tyr Ala Glu Asn Leu Tyr Phe Gln Gly
1               5                   10                  15

Gly Leu Asn Asp Ile Phe Glu Ala Gln Lys Ile Glu Trp His Glu
            20                  25                  30

<210>  4
<211>  20
<212>  DNA
<213>  Artificial Sequence

<220>
<223>  The sequence of Brd4-N-ATF sgRNA

<400>  4
tgggatcact agcatgtcta                                                   20

<210>  5
<211>  20
<212>  DNA
<213>  Artificial Sequence

<220>
<223>  The sequence of Brd4-C-FTA sgRNA

<400>  5
aatctttttt gagagcaccc                                                   20

<210>  6
<211>  20
<212>  DNA
<213>  Artificial Sequence

<220>
<223>  The sequence of Brd4-C-HTA sgRNA

<400>  6
aatctttttt gagagcaccc                                                   20

<210>  7
<211>  132
<212>  DNA
<213>  Artificial Sequence

<220>
<223>  The base sequence of Brd4-N-ATF label

<400>  7
ggtctgaacg acatcttcga ggctcagaaa atcgaatggc acgaagagaa cctgtacttc      60

cagggcgact acaaagacca tgacggtgat tataaagatc atgacatcga ctacaaggat     120

gacgatgaca ag                                                          132

<210>  8
<211>  132
<212>  DNA
<213>  Artificial Sequence

<220>
<223>  The base sequence of Brd4-C-FTA label

<400>  8
gactacaaag accatgacgg tgattataaa gatcatgaca tcgactacaa ggatgacgat      60

gacaaggaga acctgtactt ccagggcggt ctgaacgaca tcttcgaggc tcagaaaatc     120

gaatggcacg aa                                                         132


<210>  9
<211>  93
<212>  DNA
<213>  Artificial Sequence

<220>
<223>  The base sequence of Brd4-C-HTA label

<400>  9
tatccgtatg atgtgccgga ttatgcggag aacctgtact tccagggcgg tctgaacgac      60

atcttcgagg ctcagaaaat cgaatggcac gaa                                   93


<210>  10
<211>  20
<212>  DNA
<213>  Artificial Sequence

<220>
<223>  Brd4-gN-F4 (the sequence of the left and right homologous arm
       amplification primer of Brd4-N-ATF)

<400>  10
ggctgccatg tagttccagt                                                  20


<210>  11
<211>  20
<212>  DNA
<213>  Artificial Sequence

<220>
<223>  Brd4-gN-R4 (the sequence of the left and right homologous arm
       amplification primer of Brd4-N-ATF)

<400>  11
ggcctgcgtt gtagacattt                                                  20


<210>  12
<211>  23
<212>  DNA
<213>  Artificial Sequence

<220>
<223>  Brd4-gN-F6 (the sequence of the left and right homologous arm
      amplification primer of Brd4-N-ATF)

<400>  12
ccaagcccag atagatggct agt                                              23


<210>  13
<211>  23
<212>  DNA
<213>  Artificial Sequence

<220>
<223>  Brd4-gN-R2 (the sequence of the left and right homologous arm
      amplification primer of Brd4-N-ATF)

<400>  13
aaccattcac tggggttcag att                                              23


<210>  14
<211>  25
<212>  DNA
<213>  Artificial Sequence

<220>
<223>  Brd4-gC-F (The sequence of the left and right homologous arm
      amplification primer of Brd4-C-FTA)

<400>  14
gaggagaaga ttcactcacc aatca                                            25


<210>  15
<211>  25
<212>  DNA
<213>  Artificial Sequence

<220>
<223>  Brd4-gC-R (The sequence of the left and right homologous arm
      amplification primer of Brd4-C-FTA)

<400>  15
caagccagaa tacctagttg cttca                                            25


<210>  16
<211>  25
<212>  DNA
<213>  Artificial Sequence

<220>
<223>  Brd4-gC-F (The sequence of the left and right homologous arm
      amplification primer of Brd4-C- HTA)

<400>  16
gaggagaaga ttcactcacc aatca                                            25


<210>  17
<211>  25
<212>  DNA

<213>  Artificial Sequence

<220>
<223>  Brd4-gC-R (The sequence of the left and right homologous arm
       amplification primer of Brd4-C- HTA)

<400>  17
caagccagaa tacctagttg cttca                                          25


<210>  18
<211>  20
<212>  DNA
<213>  Artificial Sequence

<220>
<223>  FTA-F1 (The sequence of Brd4-N-ATF realtime PCR amplification
       primer)

<400>  18
caaggatgac gatgacaagg                                                20


<210>  19
<211>  20
<212>  DNA
<213>  Artificial Sequence

<220>
<223>  FTA-R1(The sequence of Brd4-N-ATF realtime PCR amplification
       primer)

<400>  19
ctgagcctcg aagatgtcgt                                                20


<210>  20
<211>  20
<212>  DNA
<213>  Artificial Sequence

<220>
<223>  FTA-F2(The sequence of Brd4-N-ATF realtime PCR amplification
       primer)

<400>  20
caaggatgac gatgacaagg                                                20


<210>  21
<211>  20
<212>  DNA
<213>  Artificial Sequence

<220>
<223>  FTA-R2(The sequence of Brd4-N-ATF realtime PCR amplification
       primer)

<400>  21
ttcgtgccat tcgattttct                                                20


<210>  22

<211>  20
<212>  DNA
<213>  Artificial Sequence

<220>
<223>  ATF-F1(The sequence of Brd4-N-ATF realtime PCR amplification
       primer)

<400>  22
cttcgaggct cagaaaatcg                                                   20


<210>  23
<211>  20
<212>  DNA
<213>  Artificial Sequence

<220>
<223>  ATF-R1(The sequence of Brd4-N-ATF realtime PCR amplification
       primer)

<400>  23
gtctttgtag tcgccctgga                                                   20


<210>  24
<211>  20
<212>  DNA
<213>  Artificial Sequence

<220>
<223>  ATF-F2(The sequence of Brd4-N-ATF realtime PCR amplification
       primer)

<400>  24
aaatcgaatg gcacgaagag                                                   20


<210>  25
<211>  20
<212>  DNA
<213>  Artificial Sequence

<220>
<223>  ATF-R2(The sequence of Brd4-N-ATF realtime PCR amplification
       primer)

<400>  25
gtctttgtag tcgccctgga                                                   20


<210>  26
<211>  20
<212>  DNA
<213>  Artificial Sequence

<220>
<223>  HTA-F2(The sequence of Brd4-N-ATF realtime PCR amplification
       primer)

<400>  26
gcggagaacc tgtacttcca                                                   20

<210>  27
<211>  20
<212>  DNA
<213>  Artificial Sequence

<220>
<223>  HTA-R2(The sequence of Brd4-N-ATF realtime PCR amplification
       primer)

<400>  27
ttcgtgccat tcgattttct                                                   20


<210>  28
<211>  20
<212>  DNA
<213>  Artificial Sequence

<220>
<223>  HTA-F3(The sequence of Brd4-N-ATF realtime PCR amplification
       primer)

<400>  28
tatgatgtgc cggattatgc                                                   20


<210>  29
<211>  20
<212>  DNA
<213>  Artificial Sequence

<220>
<223>  HTA-R3(The sequence of Brd4-N-ATF realtime PCR amplification
       primer)

<400>  29
ctgagcctcg aagatgtcgt                                                   20


<210>  30
<211>  20
<212>  DNA
<213>  Artificial Sequence

<220>
<223>  Brd4-gN-CN-F(The sequence of Brd4-N-ATF realtime PCR
       amplification primer)

<400>  30
gtccacagtg gcctttcaat                                                   20


<210>  31
<211>  20
<212>  DNA
<213>  Artificial Sequence

<220>
<223>  Brd4-gN-CN-R(The sequence of Brd4-N-ATF realtime PCR
       amplification primer)

<400>  31

agctgtcttc agaccctcca                                                    20

<210>  32
<211>  20
<212>  DNA
<213>  Artificial Sequence

<220>
<223>  Brd4-gC-CN-F1(The sequence of Brd4-N-ATF realtime PCR
       amplification primer)

<400>  32
ttgccttgaa cagaccctct                                                    20

<210>  33
<211>  20
<212>  DNA
<213>  Artificial Sequence

<220>
<223>  Brd4-gC-CN-R1(The sequence of Brd4-N-ATF realtime PCR
       amplification primer)

<400>  33
acacaggtgg gaaggaactg                                                    20

<210>  34
<211>  20
<212>  DNA
<213>  Artificial Sequence

<220>
<223>  Brd4-gC-CN-F2(The sequence of Brd4-N-ATF realtime PCR
       amplification primer)

<400>  34
acagaagcag gagccaaaaa                                                    20

<210>  35
<211>  20
<212>  DNA
<213>  Artificial Sequence

<220>
<223>  Brd4-gC-CN-R2(The sequence of Brd4-N-ATF realtime PCR
       amplification primer)

<400>  35
aaaggtcaag aggcaggtga                                                    20

<210>  36
<211>  20
<212>  DNA
<213>  Artificial Sequence

<220>
<223>  The sequence of Atad2b-C-HTA sgRNA

<400>  36
actcagcatg agaagttcat                                                   20


<210>  37
<211>  20
<212>  DNA
<213>  Artificial Sequence

<220>
<223>  The sequence of Baz2b-C-HTA sgRNA

<400>  37
acaacttcag ctcactttga                                                   20


<210>  38
<211>  20
<212>  DNA
<213>  Artificial Sequence

<220>
<223>  The sequence of Brd3-C-HTA sgRNA

<400>  38
actcagagtg aactcggact                                                   20


<210>  39
<211>  20
<212>  DNA
<213>  Artificial Sequence

<220>
<223>  The sequence of Cecr2-C-HTA sgRNA

<400>  39
tgtactttca gagctagtcc                                                   20


<210>  40
<211>  20
<212>  DNA
<213>  Artificial Sequence

<220>
<223>  The sequence of Baz1b-C-HTA sgRNA

<400>  40
cggagacaga agaagtaaag                                                   20


<210>  41
<211>  20
<212>  DNA
<213>  Artificial Sequence

<220>
<223>  The sequence of Pbrm1-C-HTA sgRNA

<400>  41
gatgtgatta aacattttct                                                   20

<210>  42
<211>  20
<212>  DNA
<213>  Artificial Sequence

<220>
<223>  Atad2b-gC-F(The sequence of the left and right homologous arm
       amplification primer of Atad2b-C-HTA)

<400>  42
aggagccgcc agaaatgaaa                                                    20


<210>  43
<211>  20
<212>  DNA
<213>  Artificial Sequence

<220>
<223>  Atad2b-gC-R(The sequence of the left and right homologous arm
       amplification primer of Atad2b-C-HTA)

<400>  43
tttgcctctt tgcaactgcc                                                    20


<210>  44
<211>  20
<212>  DNA
<213>  Artificial Sequence

<220>
<223>  Baz2b-gC-F(The sequence of the left and right homologous arm
       amplification primer of Baz2b-C-HTA)

<400>  44
cgggcgtgac tcgtctatta                                                    20


<210>  45
<211>  20
<212>  DNA
<213>  Artificial Sequence

<220>
<223>  Baz2b-gC-R(The sequence of the left and right homologous arm
       amplification primer of Baz2b-C-HTA)

<400>  45
tctatgtgcc tccaacaggc                                                    20


<210>  46
<211>  20
<212>  DNA
<213>  Artificial Sequence

<220>
<223>  Brd3-gC-F(The sequence of the left and right homologous arm
       amplification primer of Brd3-C-HTA)

<400>  46

cagatgacag gtcgtagccc                                                    20


<210>  47
<211>  20
<212>  DNA
<213>  Artificial Sequence

<220>
<223>  Brd3-gC-R(The sequence of the left and right homologous arm
       amplification primer of Brd3-C-HTA)

<400>  47
gaacagggac ccgtgtcaaa                                                    20


<210>  48
<211>  20
<212>  DNA
<213>  Artificial Sequence

<220>
<223>  Cecr2-gC-F(The sequence of the left and right homologous arm
       amplification primer of Cecr2-C-HTA)

<400>  48
aacagttgcc accgcataag                                                    20


<210>  49
<211>  22
<212>  DNA
<213>  Artificial Sequence

<220>
<223>  Cecr2-gC-R(The sequence of the left and right homologous arm
       amplification primer of Cecr2-C-HTA)

<400>  49
gagggaaaac tccattgacc cc                                                 22


<210>  50
<211>  20
<212>  DNA
<213>  Artificial Sequence

<220>
<223>  Baz1b-gC-F(The sequence of the left and right homologous arm
       amplification primer of Baz1b-C-HTA)

<400>  50
ttgatcgcgg catcacttca                                                    20


<210>  51
<211>  20
<212>  DNA
<213>  Artificial Sequence

<220>
<223>  Baz1b-gC-R(The sequence of the left and right homologous arm
       amplification primer of Baz1b-C-HTA)

<400>  51
gatgctgaca ctccgctaga                                                    20


<210>  52
<211>  20
<212>  DNA
<213>  Artificial Sequence

<220>
<223>  Pbrm1-gC-F(The sequence of the left and right homologous arm
       amplification primer of Pbrm1-C-HTA)

<400>  52
agtctgccaa gctgttcact                                                    20


<210>  53
<211>  20
<212>  DNA
<213>  Artificial Sequence

<220>
<223>  Pbrm1-gC-R(The sequence of the left and right homologous arm
       amplification primer of Pbrm1-C-HTA)

<400>  53
accacccaag caggttcaaa                                                    20


<210>  54
<211>  20
<212>  DNA
<213>  Artificial Sequence

<220>
<223>  The sequence of Phf7-N-Flag sgRNA

<400>  54
ttctagatag gaaggacaga                                                    20


<210>  55
<211>  23
<212>  DNA
<213>  Artificial Sequence

<220>
<223>  Phf7-gN-F(The sequence of the left and right homologous arm
       amplification primer of Phf7-N-Flag)

<400>  55
aaagtagatc cccgtgggga cac                                                23


<210>  56
<211>  22
<212>  DNA
<213>  Artificial Sequence

<220>
<223>  Phf7-gN-R(The sequence of the left and right homologous arm

amplification primer of Phf7-N-Flag)

<400>  56
gtttgtacgg ctgacaagga gc                                                22


<210>  57
<211>  20
<212>  DNA
<213>  Artificial Sequence

<220>
<223>  The sequence of Hspg2-C-Flag sgRNA

<400>  57
tcataggcac ccacctgcct                                                   20


<210>  58
<211>  20
<212>  DNA
<213>  Artificial Sequence

<220>
<223>  Hspg2-gC-F(The sequence of the left and right homologous arm
       amplification primer of Hspg2-C-Flag)

<400>  58
gtcctaatgt ggcggtcaac                                                   20


<210>  59
<211>  21
<212>  DNA
<213>  Artificial Sequence

<220>
<223>  Hspg2-gC-R(The sequence of the left and right homologous arm
       amplification primer of Hspg2-C-Flag)

<400>  59
acctcttcca gtccccttgt c                                                 21

## Description

### Technical Field

The present disclosure relates to the field of biology, specifically to the field of proteomics research, and more specifically to a high-throughput protein analysis method and a suitable library thereof.

### Background

At present, more than 26,000 functional genes encoding proteins have been discovered and located through the Human Genome Project. Among the functional genes, the functions of 42% of the genes are still unknown. In the known genes, enzymes account for 10.28%, nucleases account for 7.5%, signal transduction accounts for 12.2%, transcription factors account for 6.0%, signal molecules account for 1.2%, receptor molecules account for 5.3%, and selective regulatory molecules account for 3.2%, etc. Discovering and understanding the role of the functional genes is of great significance for understanding the life and the screening of new drugs. In the study of protein functions, the preparation of corresponding antibodies has become an indispensable task, but the acquisition of the antibodies has the following problems: 1) the preparation is complicated and the cost is high; 2) many proteins lack antibodies; 3) the specificity of antibodies from different sources and different purposes of research lead to a wide variety of antibodies of the same protein, and different antibodies needs to be selected for different experiments; 4) many antibodies are incompetent when proteins are studied in cells and in vivo; 5) different antibody preparation batches of a same antibody company may lead to possible natural differences and so on. The problems have led to a great waste of scientific research time and funds, which has brought great trouble to researchers and restricted the research process.

The binding of an ovum to a sperm forms a pluripotent fertilized ovum that begins a life. A life individual with more than 200 different somatic cells is ultimately formed by embryonic development, and the process is extremely complicated. In the development process from the fertilized ovum to a biological individual, the cell is always faced with choice: to maintain the existing identity and status or to transform into another identity and status. The maintenance and change of cell identity and status are controlled by the intrinsic genetic factors of the cell itself and also regulated by the environmental factors surrounding the cell. The interaction of intracellular and extracellular factors makes the fate of the cell variable and transformational. After the birth of the life individual, it will undergo a process of growth, maturity and aging, and the material basis of all the changes is biomacromolecules including proteins. However, how do the biomacromolecules function in life activities? How do the biomacromolecules act synergistically? Revealing the problems will help to understand life and provide theoretical support for further regulating life and avoiding diseases. However, current research on the biomacromolecules lacks a system suitable for in vivo, real-time and dynamic research.

### Summary

In view of the shortcomings of the existing technology, a first aspect of the present disclosure is to provide a high-throughput protein analysis method, including: using a tagged semi-cloned mouse library to perform parallel indicator analysis on a plurality of different target proteins of interest with one or several tag protein antibodies. In the tagged mouse library, each semi-cloned mouse is a semi-cloned mouse obtained by culturing after injecting an androgenetic haploid embryonic stem cell into an ovum, or a sexually propagated progeny thereof. The androgenetic haploid embryonic stem cell contains a gene that expresses a fusion protein of a target protein of interest and a tag protein, and the semi-cloned mouse can express the fusion protein of the target protein of interest and the tag protein.

A second aspect of the present disclosure is to further provide a tagged semi-cloned mouse library suitable for the aforementioned high-throughput protein analysis method and a method for constructing the same.

In the tagged semi-cloned mouse library of the present disclosure, the target proteins of interest expressed by each semi-cloned mouse are all expressed in fusion with the tag proteins, each semi-cloned mouse is a semi-cloned mouse obtained by culturing after injecting an androgenetic haploid embryonic stem cell into an ovum, or a sexually propagated progeny thereof, and the androgenetic haploid embryonic stem cell contains a gene that expresses a fusion protein of the target protein of interest and the tag protein.

The tagged semi-cloned mouse library of the present disclosure or the semi-cloned mouse from the library can be used in the fields of protein analysis, protein function research or drug research.

A third aspect of the present disclosure is to further provide a tagged androgenetic haploid embryonic stem cell library suitable for the aforementioned high-throughput protein analysis method and a method for constructing the same.

In the tagged androgenetic haploid embryonic stem cell library of the present disclosure, each androgenetic haploid embryonic stem cell contains a gene that expresses a fusion protein of a target protein of interest and a tag protein, and the semi-cloned mouse obtained by culturing after injecting the androgenetic haploid embryonic stem cell into an ovum can express the fusion protein of the target protein of interest and the tag protein.

The tagged androgenetic haploid embryonic stem cell library of the present disclosure or the androgenetic haploid embryonic stem cell from the library can be used in the fields of protein analysis, protein function research, and drug research.

The present disclosure can obtain the following beneficial effects:
a) Scientific research of proteins is greatly simplified, the complicated preparation process of antibodies is avoided, no expensive antibodies are needed, and the research problem of target proteins that the antibodies are difficult to prepare is solved. The conventional "tag" antibodies are utilized to easily achieve proteomics analysis and protein interaction network analysis, easily screen drug targets, and provide superior analysis schemes and low analysis costs for disease diagnosis and treatment.
b) The application of the present disclosure can allow the study of proteins to be extended from the cellular level to various stages of development and to various tissues and organs of an adult body. By adopting the present disclosure, in vivo real-time dynamic qualitative and quantitative observation is realized, an interaction network between intracellular proteins or RNA molecules is revealed, expression profiles and physiological functions of unknown proteins are explored, and whole-process monitoring of individual development is realized, etc.
c) The tag preparation with a same standard and the application of a same antibody can improve the consistency of a research system, and greatly improve the credibility of results. The present disclosure has the characteristics of low cost, high efficiency and large scale.
d) The tagged protein-coding genes of interest can be all stored in the form of cells, the tagged androgenetic haploid embryonic stem cell library is established, when necessary, the mouse can be obtained in one step by ovum injection, which greatly saves the cost of animal breeding and the like. Compared with the traditional protein overexpression research method, the present disclosure also greatly reduces the development and development time.

### Brief Description of the Drawings

Fig. 1: The schematic diagram of Brd4 endogenous genome labeling of TAP-tag
Fig. 1A: The schematic diagram of Brd4 mouse genome and three isoform thereof
Fig. 1B: The schematic diagram of TAP labeling on C-terminal or N-terminal of full-length protein isoform 3 of Brd4.
Fig. 1C: The schematic diagram of TAP-labeled Brd4 C-terminal or N-terminal corresponding to isoform 1, 2 and 3
Fig. 2: The sequence of Brd4-N-ATF label
Fig. 3: The sequence of Brd4-C-FTA label
Fig. 4: The sequence of Brd4-C-HTA label
Fig. 5: Detection of TAP-tag-labeled Brd4 protein expression level
Fig. 6: Localization detection of TAP-tag-labeled Brd4 in cells
Fig. 6A: Immunofluorescence assay image of Brd4-C-HTA-labeled androgenetic haploid embryonic stem cells and the corresponding ES cell line established after ICAHCI (sample with #)
Fig. 6B: Immunofluorescence assay image of Brd4-N-ATF-labeled and Brd4-C-FTA-labeled androgenetic haploid embryonic stem cells and the corresponding ES cell lines established after ICAHCI (sample with #)
Fig. 7: Co-IP binding protein detection of TAP-tag-labeled Brd4
Fig. 7A: Co-IP detecting result of NC and Brd4-N-ATF androgenetic haploid embryonic stem cells
Fig. 7B: Co-IP detecting result of Brd4-C-FTA, Brd4-N-ATF and Brd4-C-HTA androgenetic haploid embryonic stem cells
Fig. 8: Protein expression level detection of TAP-tag-labeled bromodomain genes
Fig.8A: Protein expression level detection result of Atad2b, Baz2b, Brd3 and Cecr2 in the C-HTA-tagged bromodomain genes in the androgenetic haploid stem cell line DKO-AG-haESCs
Fig.8B: Protein expression level detection result of Bazlb and Pbrm1 in the C-HTA-tagged bromodomain genes in the androgenetic haploid stem cell line DKO-AG-haESCs
Fig. 9: Investigation of the binding site of Phf7 in the genome of Phf7 N-terminal KI Flag tag mouse
Fig. 9A: The schematic diagram of 3×Flag sequence inserted at the N-terminal of a Phf7 endogenous genome of the androgenetic haploid embryonic stem cell
Fig. 9B: The detection results of a Phf7-KI-Flag heterozygous mouse F0 obtained by ICAHCI injection, and a Phf7-KI-Flag homozygous male mouse obtained by mating between F1 heterozygous mice
Fig. 9C: The detection result of the expression of Phf7-Flag in different germ cells isolated from the Phf7-KI-Flag homozygous male mice
Fig. 9D: The detection result of the expression of Phf7-Flag in the germ cells of the Phf7-KI-Flag homozygous male mice by Co-IP
Fig. 9E: Chip-seq detection on Phf7 by using the Flag antibody, and the comparison with the results of H3K4me3 chip-seq and ubH2A Chip-seq on the promoter/exon/intron/intergenic region enrichment situation
Fig. 9F: The overlap ratio Venn diagram of the peaks of Phf7 chip-seq and H3K4me3 chip-seq binding regions
Fig. 9G: Signal distribution Heatmap of ubH2A in H3K4me3&Phf7 common, H3K4me3 unique, and Phf7 unique results
Fig. 9H: The signal result value of ubH2A
Fig. 10: The protein detection of Hspg2 C-terminal KI-Flag mouse embryos at embryonic E15.5 days

### Detailed Description of the Preferred Embodiments

The present disclosure provides a high-throughput protein analysis method, including: using a tagged semi-cloned mouse library to perform parallel indicator analysis on a plurality of different target proteins of interest with one or several tag protein antibodies. In the tagged semi-cloned mouse library, each semi-cloned mouse is a semi-cloned mouse obtained by culturing after injecting an androgenetic haploid embryonic stem cell into an ovum, or a sexually propagated progeny thereof, the androgenetic haploid embryonic stem cell contains a gene that expresses a fusion protein of a target protein of interest and a tag protein, and the semi-cloned mouse can express the fusion protein of the target protein of interest and the tag protein.

It is called a high-throughput protein analysis method because it uses the tagged semi-cloned mouse library, and can perform simultaneous parallel research on a plurality of target proteins of interest in the library by only needing to use a limited number of universal tag protein antibodies corresponding to tags in the library. Not only does it not require the preparation of antibodies to the target protein of interest, but the same operation procedure can be used to perform simultaneously in vivo study of a plurality of target proteins of interest. This is different from the existing research method that needs to prepare antibodies of the target proteins of interest, the scientific research of proteins is greatly simplified, and the research efficiency is improved. The protein analysis method of the present disclosure does not need to prepare or use antibodies of target proteins of interest. It has very obvious advantages, especially for the study on target proteins that the antibodies are difficult to prepare or only have very expensive antibodies. The method changes the conventional in vivo research idea of proteins, and provides great convenience for drug screening, drug action mechanism analysis, drug metabolism and other researches. Due to that the same antibody is used for research, the antibody-antigen binding affinity is also consistent. Compared with the situation that different target protein antibodies are utilized for different target proteins of interest, when parallel comparison is performed, the tag protein antibody research is matured, the antibody of the present disclosure is stable in both sensitivity and specificity, and is stronger in reference.

The androgenetic haploid embryonic stem cell of the present disclosure has the self-replication ability and pluripotency of stem cells, and can replace the sperm to bind with an oocyte to support the complete development of an embryo.

The semi-cloned mouse of the present disclosure may be in the morphologies of various stages after injecting the androgenetic haploid embryonic stem cell into the ovum, including the morphology of the diploid embryonic stem cell, the morphology of the embryonic stage, and the morphology of each development and growth stage after the newborn.

Indicator analysis performed on the target proteins of interest using tag protein antibodies mainly utilizes the antigen-antibody binding property between the tag protein antibodies and the tag proteins. Due to the fusion expression of the target protein and the tag protein, the tag protein antibodies can indicate the target proteins of interest.

The existing immunoassay test methods utilizing an antigen-antibody specific binding reaction are all suitable for use in the indicator analysis performed on the target proteins of interest using the tag protein antibodies in the present disclosure, including but not limited to: western blot, immunofluorescence assay (IF), immunoprecipitation (IP), co-immunoprecipitation (Co-IP), chromatin immunoprecipitation (Chip-seq), RNA immunoprecipitation (RIP), cross-linked immunoprecipitation (CLIP), mass spectrometry MS, Elisa, tandem affinity purification technology, fluorescence resonance energy transfer technology, fusion reporter gene localization, etc. When specific analytical experiments are performed, analysis samples may be taken from physiological slice samples, tissue samples, body fluid samples, in vitro cell samples, organ samples, etc. from semi-cloned mice of various morphologies.

The protein analysis method of the present disclosure includes, but is not limited to, analysis of protein expression, protein spatiotemporal localization, protein-protein interaction, protein metabolism, protein DNA binding region, protein and RNA binding region, and the like.

Specifically, the expression situation of proteins can be determined by the western blot and the Elisa; the spatiotemporal localization of proteins can be performed by the immunofluorescence assay and the fusion reporter gene localization; the protein-protein interaction can be analyzed by the co-immunoprecipitation technology, the tandem affinity purification technology, the fluorescence resonance energy transfer technology, and the co-immunoprecipitation-mass spectrometry (Co-IP-MS); the protein metabolism is analyzed by the nuclear magnetic resonance (NMR), the mass spectrometry (MS), chromatography (HPLC, GC) and chromatography-mass spectrometry technology; the protein DNA binding region is analyzed by Chip-seq; the protein and RNA specific binding region is analyzed by RIP, CLIP, and RNA Western blot, and the physiological metabolic network of the proteins is comprehensively analyzed and studied by the above systems.

The present disclosure can be utilized to analyze samples of various growth stages and various tissues from semi-cloned mice to understand the expression of proteins in various tissues of mice, the expression at a specific growth stage, or the expression at a certain growth stage. Therefore, it is considered that the protein analysis method of the present disclosure is suitable for in vivo, real-time, and dynamic analysis.

It should be understood that the protein analysis method of the present disclosure is not intended for the diagnosis or treatment of a disease.

The protein analysis method of the present disclosure can be realized by utilizing a tagged semi-cloned mouse library. In the tagged semi-cloned mouse library of the present disclosure, the target protein of interest expressed by each of the semi-cloned mice is expressed in fusion with the tag protein. Each semi-cloned mouse may be a semi-cloned mouse obtained by culturing after injecting an androgenetic haploid embryonic stem cell into an ovum, or may also be a sexually propagated progeny thereof. The androgenetic haploid embryonic stem cell used for constructing the semi-cloned mouse should contain a gene that expresses a fusion protein of the target protein of interest and the tag protein.

The tagged androgenetic haploid embryonic stem cell may be taken as a donor of ICAHCI, a semi-cloned embryo is obtained by an ICAHCI method, and the semi-cloned embryo can be further cultured in a suitable mother mouse by an embryo transfer method to obtain a semi-cloned mouse.

Based on the constructed tagged semi-cloned mouse library, the protein in vivo analysis can be realized conveniently and quickly by only needing to select a semi-cloned mouse that can perform fusion expression on the fusion protein of the target protein to be studied and the tag protein.

Since the semi-cloned mice are costly to breed and need to occupy a lot of space, the protein analysis method of the present disclosure more preferably utilizes a tagged androgenetic haploid embryonic stem cell library to construct a semi-cloned mouse or a semi-cloned mouse library. Based on the optimized androgenetic haploid embryonic stem cell technology, the androgenetic haploid embryonic stem cells can still support the stable acquisition of semi-cloned mice after multiple rounds of in vitro genetic manipulation and long-term in vitro culture. Based on the constructed tagged androgenetic haploid embryonic stem cell library, the androgenetic haploid embryo stem cells suitable for expressing the fusion protein of the target protein and the tag protein only need to be selected from the library to be injected into the ovum before protein analysis, and it takes only one month to obtain the desired semi-cloned mouse or semi-cloned mouse library. The preparation time is short, the efficiency is high, the cage sites and time for breeding the mice are substantially saved, and the cost is greatly reduced. The tagged androgenetic haploid embryonic stem cell library is stored in the form of cells, and the mice can be obtained by ovum injection when needed, which greatly reduces the cost of animal breed conservation.

According to the purpose of research and development, the type of the target proteins of interest expressed in fusion with the tag proteins in the tagged semi-cloned mouse library or the tagged androgenetic haploid embryonic stem cell library is confirmed to constitute a target protein combination of interest. The tagged androgenetic haploid embryonic stem cells or tagged semi-cloned mice that can express the fusion protein of the target protein of interest and the tag protein in the target protein combination of interest are combined to constitute a tagged androgenetic haploid embryonic stem cell library or a tagged semi-cloned mouse library.

The selection of each target protein of interest in the target protein combination of interest can be set as desired. For example, the members of the target protein combination of interest are determined according to domain classification, functional classification, localization classification, signal pathway classification, disease pathway classification, and the like. Domain classification includes, but is not limited to, bromodomain family, death-domain family, PHD finger family, POU domain family, ring finger family, SET domain family, and the like. Functional classification includes, but is not limited to, cell adhesion, RNA binding, DNA repair, cell surface receptors, cytokines, cytokine receptors, transcription factors, inflammation-related factors, kinases, lipid transport metabolism-related factors, stress-related factors, apoptosis, nuclear receptors, cell cycle regulatory factors, heat shock proteins, growth factors, cell migration, and the like. Localization classification includes, but is not limited to, cytoplasm, nucleoli, nuclear membranes, centrosomes, Golgi apparatus, endoplasmic reticulums, mitochondria, ribosomes, cell membranes, lysosomes, and the like. Signal pathway classification includes, but is not limited to, Caspase family, IAP family, TRAF family, TNF receptor family, TNF ligand family, P53 signal pathway, DNA loss response pathway, cell cycle arrest pathway, Notch signal pathway, small GTPase protein signal pathway, Wnt signal pathway, and the like. Disease pathway classification includes, but is not limited to, cancer, immune system diseases, neurodegenerative diseases, circulation system diseases, metabolic disorder, infectious disease circulation system diseases, and the like.

The construction of the tagged androgenetic haploid embryonic stem cell may include the following steps:
1) Genetic modification was performed on the androgenetic haploid embryonic stem cell to contain a gene that expresses a fusion protein of each target protein of interest and a tag protein.
2) The androgenetic haploid embryonic stem cell that can express the fusion protein of the target protein of interest and the tag protein was screened out.
3) Breed conservation and library construction were performed on primary cells of the screened androgenetic haploid embryonic stem cells or passage haploid cells thereof to obtain a tagged androgenetic haploid embryonic stem cell library.

In step 1), genetic modification can be performed on the androgenetic haploid embryonic stem cells by using the existing technology. The genetic modification of the present disclosure may be introducing a tag protein gene in situ into a target protein of interest-coding gene already existing in the mouse androgenetic haploid embryonic stem cells; or introducing an exogenous target protein of interest-coding gene into the mouse androgenetic haploid embryonic stem cells and then introducing a tag protein gene in situ into the exogenous target protein of interest-coding gene; or directly introducing a tagged exogenous target protein of interest-coding gene into the mouse androgenetic haploid embryonic stem cells. Genetic modification can be accomplished using the existing gene targeting, homologous recombination and other technologies, including but not limited to, genetic manipulations based on ZFN (zinc finger nuclease), TALEN (transcriptional activation-like effector nuclease), and CRISPR/Cas9 (clustered regularly interspaced short palindromic repeat), and the like. In a preferred embodiment of the present disclosure, gene targeting is performed on the androgenetic haploid embryonic stem cells to introduce a tag protein gene using a CRISPR-Cas9 technology-mediated gene-editing technology.

In step 2), PCR can be used for genotype identification to screen out the androgenetic haploid embryonic stem cells that can express the fusion protein of the target protein of interest and the tag protein. A plurality of pairs of primers is designed to determine the genotype according to the particular situation to be identified. The androgenetic haploid embryonic stem cells, which are correctly sequenced, can also be subjected to western-blot assay using tag protein antibodies to screen out the androgenetic haploid embryonic stem cells that can express the fusion protein of the target protein of interest and the tag protein.

In step 3), the passage and breed conservation of the androgenetic haploid embryonic stem cells can be carried out by the conventional cell passage and breed conservation methods. Haploid cells can be collected by flow cytometry.

In order to facilitate assay of the target protein of interest using the tag protein antibody, preferably, in the fusion protein of the target protein of interest and the tag protein expressed by the androgenetic haploid embryonic stem cells or semi-cloned mice, the tag protein is completely or partially exposed to the surface of the fusion protein. Whether the tag protein can be exposed to the designed fusion protein or not can be predicted by means of related simulation software such as OMIC Tools, I-TASSER, HHpred, RaptorX, IntFOLD, NAMD (NAnoscale Molecular Dynamics) and VMD

A preferred method is to allow the tag protein to be located at the N-terminal and/or C-terminal of the target protein of interest. This method is suitable for most of the target proteins of interest. If it is found that placing the tag protein at the N-terminal or C-terminal of the target protein of interest cannot allow the antigenic determinant of the tag protein to be exposed, the tag protein can also be inserted into a suitable position in the target protein of interest so as to enable the antigenic determinant of the tag protein to be successfully exposed. For example, the related design can be carried out by using OMIC Tools, I-TASSER, HHpred, RaptorX, IntFOLD, NAMD (NAnoscale Molecular Dynamics) and VMD software. For example, when there is a signal peptide, the tag protein can be designed at the C-terminal of the target protein of interest, or the tag protein can be designed behind the N-terminal signal peptide.

The N-terminal of the target protein of interest is fused with the tag protein by inserting the tag protein gene behind an initiation codon ATG of the target protein of interest and before the target protein of interest-coding gene; the C-terminal of the target protein of interest is fused with the tag protein by inserting the tag protein gene before a termination codon of the target protein of interest and behind the target protein of interest-coding gene. When the N-terminal of the target protein of interest has a signal peptide, the tag protein gene can be inserted between a signal peptide-coding gene of the target protein of interest and the remaining coding genes of the target protein of interest.

In the present disclosure, the tag protein used may be selected from one or more of the following: Flag, HA, Green Proteins (TurboGFP, TagGFP2, mUKG, Superfolder GFP, Emerald, EGFP, Monomeric Azami Green, mWasabi, Clover, mNeonGreen, NowGFP, mClover3), Red Proteins (TagRFP, TagRFP-T, RRvT, mRuby, mRuby2, mTangerine, mApple, mStrawberry, FusionRed, mCherry, mNectarine, mRuby3, mScarlet, mScarlet-I), Cyan Proteins (ECFP, Cerulean, mCerulean3, SCFP3A, CyPet, mTurquoise, mTurquoise2, TagCFP, mTFP1, monomeric Midoriishi-Cyan, Aquamarine), Yellow Proteins (TagYFP, EYFP, Topaz, Venus, SYFP2, Citrine, Ypet, lanRFP-Δ S83, mPapaya1, mCyRFP1), Orange Proteins (Monomeric Kusabira-Orange, mOrange, mOrange2, mKO κ, mKO2), Myc, His, GST, Strep, CBP, MBP, iDimerize, ProteoTuner, Shield1, SNAP-tag, CLIP-tag, ACP-tag, MCP-tag, HaloTag, Avi-tag, TAP-tag, Lumio™ tag and the like. The selection of specific tag proteins can follow the following principle that: the fluorescence resonance energy transfer technology and the fusion reporter gene localization can select Green Proteins, Red Proteins, Cyan Proteins, Yellow Proteins, Orange Proteins, SNAP-tag, CLIP-tag, ACP- Tag, MCP-tag, Lumio™ tag, etc. according to the needs; the rapid degradation of specific regulatory proteins can select ProteoTuner according to the needs; the realization of induced protein relocation or protein-protein interactions can select iDimerize according to the needs; western blot, immunofluorescence assay (IF), co-immunoprecipitation (Co-IP), Chip-seq, mass spectrometry MS, Elisa, tandem affinity purification technology, and RNA Western blot can select one or more of Flag, HA, Myc, His, GST, Strep, CBP, MBP, HaloTag, Avi-tag, TAP-tag, etc. according to the needs. When a plurality of tag proteins is selected at a label site, the tag proteins may be directly linked to each other or may be linked by a linker peptide. In order to facilitate multiple operations, a protein or polypeptide sequence and the like which can be digested by a specific enzyme can also be linked between the tag proteins. In the specific embodiments of the present disclosure, 3×Flag-TEV-Avi, 3×Flag-TEV-Avi and HA-TEV-Avi are respectively selected as labeled proteins of bromodomain proteins.

Studies have found that knockout of H19 DMR and IG-DMR of the androgenetic haploid embryonic stem cells can solve the problem of low birth efficiency and developmental defects of the semi-cloned mice. The androgenetic haploid embryonic stem cells of the present disclosure are preferably selected from the H19 DMR and IG-DMR-knockout androgenetic haploid embryonic stem cells, i.e. DKO-AG-haESCs.

H19 DMR refers to a differentially methylated region (DMR) in an *H19*-Igf2 imprinted cluster. The specific location and sequence of H19 DMR can be determined by the existing methods such as methylation sequencing or homologous sequence analysis prediction. It is known that the human H19 DMR is located in the chromosome 11p15.5 region, and the mouse H19 DMR is located at the distal end of chromosome No. 7, between two genes of H19 and Igf2, at 2 kb to 4 kb upstream of the H19 gene. H19 DMR is in a methylated state on a paternal allele, resulting in the inability of a CTCF protein to bind to the methylated region, so that an enhancer at the downstream of H19 does not need to overcome the obstacle of CTCF, thereby enhancing the expression of upstream Igf2 and reducing the expression of H19. H19 DMR is in a demethylated state on a maternal allele, resulting in the ability of the CTCF protein to bind to the unmethylated region, so an enhancer at the downstream of H19 can only enhance the expression of H19, but cannot regulate the upstream Igf2. If the paternal H19 DMR is knocked out, then the enhancer at the downstream of H19 can up-regulate the expression of Igf2. Since the androgenetic haploid is from a paternal origin, it should theoretically be in a completely methylated state, but the study found that the methylation of the androgenetic haploid H19 DMR cultured in vitro is abnormally erased and the androgenetic haploid H19 DMR becomes in a demethylated state, resulting in abnormal up-regulation of H19 expression and down-regulation of Igf2 expression. Knockout of H19 DMR can correct the abnormal state of the up-regulation of H19 expression and down-regulation of Igf2 expression.

IG-DMR refers to a differentially methylated region (DMR) in a *Dlk-Dio3* imprinted cluster. The specific location and sequence of IG DMR can be determined by the existing methods such as methylation sequencing or homologous sequence analysis prediction. It is known that the mouse IG-DMR is located on chromosome No. 12, which is a 4.15 kb repeat sequence between Dlk1 and Gtl2 genes in the imprinted cluster, and the human IG-DMR is located on chromosome No. 14 (14q32.2). When IG-DMR is located in a paternal allele, DNA methylation occurs in this region, the gene Gtl2 and some mircroRNAs in the imprinted cluster are not expressed, but genes Rtll, Dlk1 and Dio3 are expressed. When it is in a maternal allele, this region does not undergo DNA methylation (demethylated state), so Gtl2 and some mircroRNAs are expressed, but genes Rtll, Dlk1 and Dio3 are not expressed. In androgenetic haploid (parental origin) and abnormally born SC animals, the study found that methylation of IG-DMR, which should be in a methylated state, is abnormally erased, resulting in the silencing of genes Rtll, Dlk1 and Dio3, and abnormal activation of Gtl2 and some mircroRNAs.

When protein analysis is performed using the tagged semi-cloned mouse library or tagged androgenetic haploid embryonic stem cell library, in a preferred embodiment, the tag proteins expressed in fusion with each target protein of interest are the same. In this case, a tag protein or a plurality of tag proteins may be used to label the target protein of interest, for example, the tag protein is expressed in fusion with the target protein of interest at the N-terminal or C-terminal, or different tag proteins are expressed in fusion with the target protein of interest at the N-terminal or C-terminal. When a plurality of tag proteins is used to label the target proteins of interest, it is only necessary to ensure that each target protein of interest is expressed in fusion with the same tag proteins, so that each target protein of interest can be ensured to have the same tag proteins. The same tag protein expressed in fusion with each target protein of interest can simplify the parallel analysis operation and facilitate parallel analysis.

Of course, in the tagged semi-cloned mouse library or the tagged androgenetic haploid embryonic stem cell library, the tag proteins expressed in fusion with each target protein of interest may also be different. However, since the tag proteins in the library come from a combination consisting of a limited number of tag proteins, the parallel analysis can also be performed on each target protein of interest on the basis of not preparing an antibody of the target protein of interest by only using an antibody of each tag protein in the combination consisting of the limited number of tag proteins.

By using the mice or androgenetic embryonic stem cells in the library provided in the present disclosure, not only can the target protein of interest be analyzed, but also drug research can be performed. In an embodiment in which the mice or androgenetic embryonic stem cells in the library provided in the present disclosure are applied to drug research, the mechanism of action of the drug is understood by studying the target protein of interest before and after the action of the drug. In another embodiment, a drug having a specific effect can be screened out by high throughput by the change in the expression of each target protein of interest in the mice before and after the action of the drug. In another embodiment, by constructing a tagged toxicological model animal, an in vivo study on drug metabolism is performed by detecting the change in the expression of the corresponding toxicological protein before and after the action of the drug.

Functional studies of the knockdown expression of a target protein of interest can also be performed using the mice or androgenetic embryonic stem cells in the library provided in the present disclosure. Trim21 is an E3 ubiquitinated ligase that is brought to its specific recognition epitope, i.e., an antigen, by binding to an Fc region of an antibody, to trigger a downstream protein degradation pathway, thereby specifically degrading an antigenic protein recognized by the antibody. The target protein expressed in fusion with the tag protein can be specifically, quickly and efficiently degraded by introducing the androgenetic embryonic stem cells in the library of the present disclosure into Trim21 and a tag protein-specific antibody, i.e, transiently transfecting or genomically integrating Trim21 and a tag protein-specific antibody DNA sequence. At the same time, if Trim21 and the tag protein antibody are conditionally expressed, such as the inducible promoter Tet-On/Off system-driven expression, Doxycycline/Tetracycline-mediated inducible, specific, efficient, and rapid degradation of the target protein can be achieved. An F0 generation heterozygous tag can also be knocked in the mouse in the library of the present disclosure, and then further an F2 progeny mouse in which a homozygous tag is knocked in is obtained by a mating method. Inducible degradation regulation of the target protein can be realized by enabling the homozygous F2 progeny mouse to mate with a tool mouse that the Trim21 and the tag protein antibody are expressed driven by a tissue-specific promoter or an inducible promoter Tet-On/Off system, thereby detecting the change in mouse phenotype and physiological indicators.

The embodiments of the present disclosure are described below by way of specific examples. It should be understood that the scope of the present disclosure is not limited to the specific embodiments described below; it should also be understood that the terms used in the embodiment of the present disclosure are intended to describe specific embodiments, but not to limit the scope of the present disclosure; In the description and claims of the present disclosure, unless the context clearly indicates otherwise, the singular forms "a/an", "one" and "the" include plural forms.

Unless otherwise defined, all technical and scientific terms used in the present disclosure have the same meaning as terms generally understood by those skilled in the existing technology. In addition to the specific methods, devices, and materials used in the embodiments, any method, device, and material of the existing technology, similar or equivalent to the methods, devices, and materials described in the embodiments of the present disclosure may also be used to implement the present disclosure according to the mastery to the existing technology and the description of the present disclosure by those skilled in the art.

Unless otherwise stated, the experimental methods, detection methods, and preparation methods disclosed in the present disclosure all employ conventional molecular biology, biochemistry, chromatin structure and analysis, analytical chemistry, cell culture, recombinant DNA technology in the existing technology, and conventional technologies in the related fields. These technologies are well described in the existing literature.

### Experimental materials and methods:

### 1. Construction of androgenetic haploid embryonic stem cell line

The androgenetic haploid embryonic stem cell line is constructed according to the reported method. (Yang, H., Shi, L., Wang, B.A., Liang, D., Zhong, C.,Liu, W., Nie, Y., Liu, J., Zhao, J., Gao, X. , et al. (2012). Generation of genetically modified mice by oocyte injection of androgenetic haploid embryonic stem cells. Cell 149, 605-617,).

Methods: Removing the cell nucleus of an MII ovum and injecting a corresponding sperm head into it. The mouse MII ovum was collected 14 hours after human chorionic gonadotropin (HCG) treatment and then enucleated by a Piezo needle in an HEPES-CZB culture solution containing 5 ug/ml cytochalasin B (CB). After enucleation, a single sperm head was injected into the cytoplasm of the ovum. Reconstructed embryos were cultured in a CZB culture solution for 1 hour and then transferred to an activation solution containing 1 mM Sr²⁺ for activation. After activation, all reconstructed embryos were transferred to a KSOM culture solution containing amino acid to be cultured at a temperature of 37°C under the condition of 5% CO₂. The reconstructed embryos reaching the morula or blastocyst stage after 3.5 days were planted in an ESC medium.

A reconstructed embryo zona pellucida was digested for removal by an Acid Tyrode solution. Each was transferred into wells of a 96-well plate covered with a mouse fibroblast trophoblast and cultured in an ESC medium containing 20% knockout serum replacement (KSR), 1,500 U/ml LIF, 3M CHIR99021 and 1M PD0325901. After 4 to 5 days of culture, cell clones were trypsinized and passed to a 96-well plate covered with a fresh trophoblast. The cell culture was further expanded, and passed to a 48-well plate and further to a 6-well plate, and the daily cell maintenance was only in the 6-well plate. To sort out haploid cells, after trypsinization, embryonic stem cells were washed once with PBS (GIBCO) and then had a water bath for 30 min in an ESC medium containing 15 µg/ml Hoechst 33342. Subsequently, haploid IN peak-shaped cells were sorted out by a flow sorter BD FACS AriaII and collected for subsequent culture to obtain the androgenetic haploid embryonic stem cell line.

H19 DMR and IG-DMR-knockout androgenetic haploid embryonic stem cells DKO-AG-haESCs were constructed with reference to the existing technology, as described in detail in Patent Application WO2017000302.

### 2. Construction of tagged androgenetic embryonic stem cells

Construction of CRISPR-Cas9 plasmid: the forward oligonucleotide strand and the reverse oligonucleotide strand of a synthesized sgRNA were annealed to obtain a double-stranded oligonucleotide strand (the sgRNA sequences in the present disclosure all refer to a forward oligonucleotide strand sequence of sgRNA), and then it was ligated to *pX330-mCherry* (Addgene #98750) digested with BbsI (New England Biolabs).

Construction of KI donor vector: left and right homologous arms were amplified from a genome containing a target protein of interest gene by synthesized left and right homologous arm amplification primers. If the target protein of interest is a mouse endogenous protein, the homologous arms can be amplified by using a mouse genome as a template. If the tag protein gene is a very small fragment, such as 20 to 70 bp, it can be prepared by synthesis and annealing of single-stranded DNA. If the tag protein gene is relatively long and cannot be directly synthesized, it can be constructed on a T vector or genetically synthesized, and then prepared by tag high-fidelity PCR amplification. The left and right homologous arm fragments, the tag protein gene fragment and the linearized T vector were ligated by using a seamless cloning kit to obtain the KI donor vector.

The constructed corresponding plasmid and KI donor vector were transfected into the androgenetic haploid embryonic stem cells using Lipofectamine 2000 (Life Technologies) according to the instruction. After 12 hours of transfection, haploid cells were sorted out by flow sorter (FACSAriaII, BD Biosciences), and then laid down at a lower density. After 4 to 5 days of growth, monoclones were picked for subsequent line establishment. Finally, a tagged androgenetic embryonic stem cell line was obtained by the identification of a PCR sequencing method.

The CRISPR-Cas9 technology-mediated gene-editing technology is a mature technology. The desired sgRNA Oligos can be designed online by using the CRISPR design website (http://crispr.mit.edu:8079/). A 25 to 40 bp genomic sequence near a pre-inserted tag protein site is selected for sgRNA design. Homologous arms with appropriate length (1 kb to 1.5 kb) were respectively selected at the upstream and downstream of the pre-inserted tag protein site, and amplification primers of left and right homologous arms were designed by using the online primer design website primer3 (http://primer3.ut.ee/) to amplify the left and right homologous arms to construct the KI donor vector. The androgenetic haploid embryonic stem cells were genetically edited by CRISPR-Cas9-mediated gene manipulation to obtain androgenetic haploid embryonic stem cells that can express the tagged target protein of interest.

If the target protein of interest is not derived from a mouse, the androgenetic haploid embryonic stem cells that can express the target protein of interest can also be firstly constructed by the CRISPR-Cas9 technology-mediated gene-editing technology, and further edited into the tag protein.

### 3. Construction of tagged semi-cloned mouse

Intracytoplasmic tagged AG-haESCs injection (ICAHCI):
To obtain semi-cloned (SC) embryos, the tagged AG-haESCs were treated with a medium containing 0.05 µg/ml colchicine for 8 h to synchronize cells to an M phase, followed by cytoplasmic injection. The digested AG-haESCs were washed 3 times with an HEPES-CZB culture solution, and then resuspended in a 3% (w/v) polyvinylpyrrolidone (PVP)-containing HEPES-CZB culture solution. Each cell nucleus of AG-haESCs at M phase was injected into the MII ovum by using a Piezo micromanipulator. The reconstructed embryos were firstly cultured in a CZB culture solution for 1 h and then activated with a CB-free activation solution for 5 to 6 h. After activation, all reconstructed embryos were cultured in a KSOM culture solution at a temperature of 37°C under the condition of 5% CO₂. ICAHCI embryos were cultured in the KSOM culture solution for 24 h to obtain 2-cell stage embryos.

Every 30 to 40 2-cell embryos obtained from ICAHCI were transferred to each uterus of a 0.5 dpc (0.5 days after mating) pseudopregnant ICR mouse. A mother mouse undergoes caesarean section or natural production after 19.5 days of pregnancy. After removing the fluid from the born mice, they were placed in an oxygen-containing incubator, and the surviving mouse was subsequently raised by the surrogate mother.

### 4. Western blot immunoblot analysis

Cells to be assayed were lysed with a RIPA cell lysate containing a protein inhibitor (Cell Signaling Technology), and the protein concentration was assayed by a BCA protein concentration assay kit (Beyotime); a protein sample was separated by SDS/PAGE, and then transferred by a wet method onto a nitrocellulose membrane; the membrane was blocked with 5% skim milk powder/TBST for 1 hour at room temperature; a primary antibody was hybridized at a temperature of 4 degrees overnight; TBST was used for washing three times; a secondary antibody was hybridized for 1.5 hours at room temperature; the TBST was used for washing three times; and finally, color development was carried out with a color developing solution (Tanon), and photographing was performed by using a fully automatic chemiluminescence image analysis system (Tanon).

### 5. Immunofluorescence analysis

Cells were washed once with PBS and then fixed with 4% PFA for 15 minutes at room temperature, or directly fixed with -20 degrees pre-cooled methanol for 5 minutes; the cells were washed three times with PBS; then the cells were permeabilized with 0.2% Triton X-100 for 30 minutes; then the cells were blocked in a blocking solution (PBS containing 1% BSA) for 1 hour; then the cells were incubated with a primary antibody diluted in the blocking solution at a temperature of 4 degrees overnight; the cells were washed three times with PBS; then the cells were incubated with a secondary antibody diluted in the blocking solution at room temperature for 1 hour in the dark; the cells were washed three times with PBS; then the cells were incubated with DAPI diluted in PBS at room temperature for 5 to 10 minutes in the dark; the cells were washed once with PBS; and finally, the cells were mounted with a fluorescent mounting medium and stored at a temperature of 4 degrees in the dark.

### 6. Co-IP analysis

Cells to be assayed were lysed with a TNE cell lysate containing a protein inhibitor (50 mM Tris-HCl (pH 7.5), 150 mM NaCl, 1% NP-40), and the protein concentration was assayed by a BCA protein concentration assay kit (Beyotime); the quantified cell lysate was pre-cleaned with an appropriate amount of magnetic beads at a temperature of 4 degrees for 1 hour; after the magnetic beads were removed, magnetic beads coupled with a tag antibody were added for a rotation reaction at a temperature of 4°C overnight; the magnetic beads were washed three times at a temperature of 4 degrees for 10 minutes by using the TNE cell lysate containing the protein inhibitor; an appropriate volume of 1×SDS-PAGE protein loading buffer was added and boiled in a 100°C air bath for 10 minutes. The protein samples after IP were separated by SDS/PAGE, and then were transferred onto a nitrocellulose membrane by a wet method; the membrane was blocked with 5% skim milk powder/TBST for 1 hour at room temperature; a primary antibody was hybridized at a temperature of 4 degrees overnight; TBST was used for washing three times; a secondary antibody was hybridized at room temperature for 1.5 hours; TBST was used for washing three times; and finally, color development was carried out with a color developing solution (Tanon), and photographing was performed by using a fully automatic chemiluminescence image analysis system (Tanon).

### 7. Chip-seq library construction and data analysis

Cells were fixed with formaldehyde, subjected to ultrasonication, purified by adding different antibodies and subjected to other steps. Finally, DNAs with a purified fragment size between 200 and 500 bp were used to construct a library. Each antibody corresponds to 10⁷ cells. A qualified sample library in each group of constructed libraries produced 150 bp reads by Illumina NovaSeq, and the number of reads per group is at least more than 20 megabytes. The measured data was aligned to a mouse genome mm10, and the unique aligned reads was retained; reads aligned to multiple locations were randomly selected to retain the location with the best alignment results. A protein-enriched region (Peak) was obtained by using default parameters.

### 8. Real-time quantitative PCR detection of TAP-tag-labeled genome copy number

The genomic DNA of a sample to be detected was extracted according to the genomic DNA extraction kit (Tiangen) process. Real-time quantitative PCR was accomplished with SYBR Green Realtime PCR Master Mix (TOYOBO), and a 20 µl reaction system was provided, wherein a genomic DNA template was diluted 10 times and 1 µl was added, and 40 cycles were amplified on a Bio-Rad CFX96 real-time quantitative PCR instrument. The copy number was calculated by the value of TAP-tag to the de-targeted endogenous genomic DNA value. The data was analyzed with the software of the CFX96 real-time quantitative PCR instrument.

### Embodiment 1

### Tandem affinity purification (TAP)-tag labeling of 40 bromodomain-containing mouse genes

40 bromodomain-containing mouse genes (Table 1) were labeled with Tandem affinity purification (TAP)-tag, and TAP-tag was used to capture a protein complex or DNA sequence binding to a labeled protein, thereby subsequently performing mass spectrometry MS and Chip-seq experiments. By performing MS and Chip-seq assay on 40 similar bromodomain-containing mouse genes, the specificity of the binding protein network and the DNA binding region was analyzed, and the function and division of labor of bromodomain proteins were further studied.

**Table. 1 List of 40 bromodomain-containin mouse genes**

| | **Gene Name** | **NCBI-ID** |
|---|---|---|
| 1 | Ash1l | 192195 |
| 2 | Atad2 | 70472 |
| 3 | Atad2b | 320817 |
| 4 | Baz1a | 217578 |
| 5 | Baz1b | 22385 |
| 6 | Baz2a | 116848 |
| 7 | Baz2b | 407823 |
| 8 | Bptf | 207165 |
| 9 | Brd1 | 223770 |
| 10 | Brd2 | 14312 |
| 11 | Brd3 | 67382 |
| 12 | Brd4 | 57261 |
| 13 | Brd7 | 26992 |
| 14 | Brd8 | 78656 |
| 15 | Brd9 | 105246 |
| 16 | Brdt | 114642 |
| 17 | Brpf1 | 78783 |
| 18 | Brpf3 | 268936 |
| 19 | Brwd1 | 93871 |
| 20 | Brwd3 | 382236 |
| 21 | Cecr2 | 330409 |
| 22 | Crebbp | 1291 4 |
| 23 | Ep300 | 328572 |
| 24 | Kat2a | 14534 |
| 25 | Kat2b | 1851 9 |
| 26 | Kmt2a | 214162 |
| 27 | Pbrm1 | 66923 |
| 28 | Phip | 83946 |
| 29 | Smarca2 | 67155 |
| 30 | Smarca4 | 20586 |
| 31 | Sp100 | 20684 |
| 32 | Sp110 | 109032 |
| 33 | Sp140 | 434484 |
| 34 | Taf1 | 270627 |
| 35 | Trim24 | 21848 |
| 36 | Trim28 | 21849 |
| 37 | Trim33 | 94093 |
| 38 | Trim66 | 330627 |
| 39 | Zmynd11 | 66505 |
| 40 | Zmynd8 | 228880 |

### A. TAP-tag sequence and label location selection

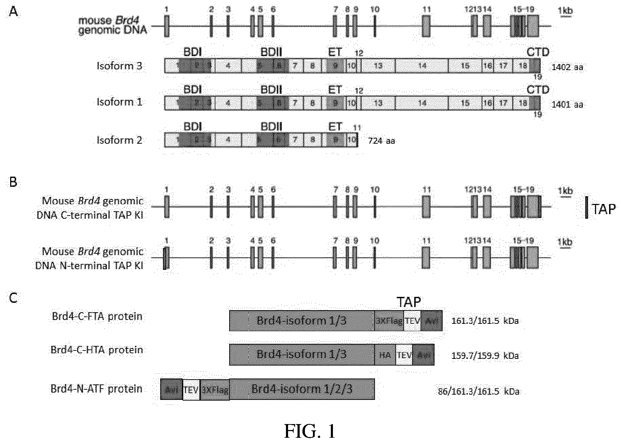

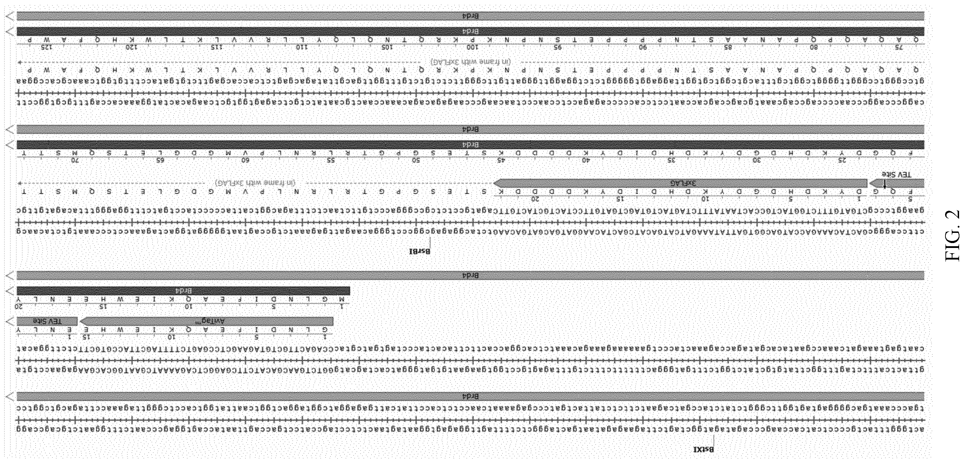

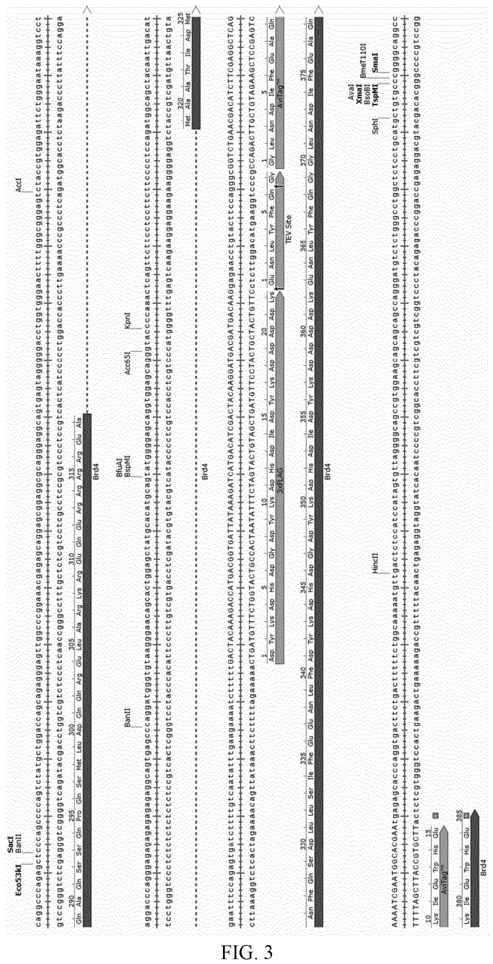

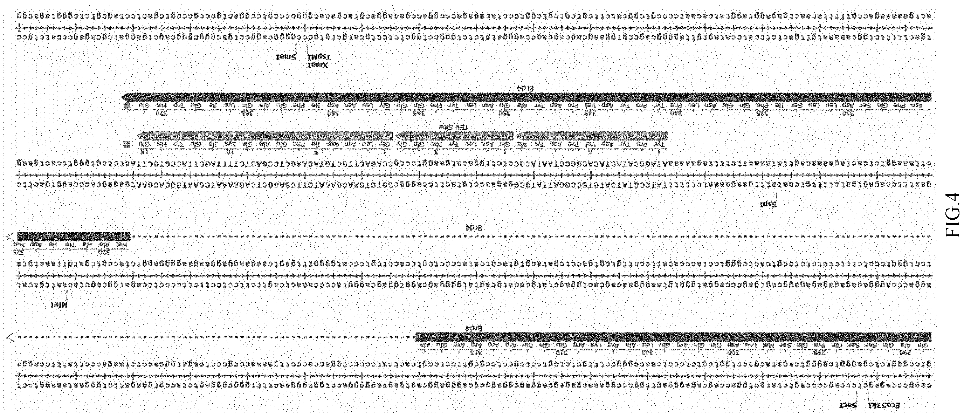

Taking a Brd4 protein as an example, Brd4 has three isoforms in total, isoforms 1, 2, and 3 express 1401, 724, and 1402 amino acids, respectively (Fig. 1A), and the full-length protein isoform 3 was selected for labeling the N-terminal and the C-terminal in its corresponding genome respectively, to detect the labeling situation of the Brd4 protein by TAP-tag (Fig. 1B). Since the C-terminals of Brd4 isoforms 1 and 3 are the same, the C-terminal TAP-tag will label the isoforms 1 and 3 at the same time; while the N-terminals of isoforms 1, 2, and 3 are the same, the N-terminal TAP-tag will label three isoforms at the same time. For the TAP-tag, the two forms 3×Flag-TEV-Avi, or HA-TEV-Avi (Fig. 1C) were selected, wherein the N-terminal was labeled with 3×Flag-TEV-Avi (N-ATF for short); the C-terminal was labeled with 3×Flag-TEV-Avi (C-FTA for short) or HA-TEV-Avi (C-HTA for short). The sequences of specific labels (see Fig.2, 3, and 4) are shown below:

The amino acid sequence of Brd4-N-ATF label (SEQ ID NO:1):
GLNDIFEAQKIEWHEENLYFQGDYKDHDGDYKDHDIDYKDDDDK

The amino acid sequence of Brd4-C-FTA label (SEQ ID NO:2):
DYKDHDGDYKDHDIDYKDDDDKENLYFQGGLNDIFEAQKIEWHE

The amino acid sequence of Brd4-C-HTA label (SEQ ID NO:3):
YPYDVPDYAENLYFQGGLNDIFEAQKIEWHE
**B. Brd4 genome labeling of TAP-tag**

According to the experimental method described in the above 2, Brd4-N-ATF, Brd4-C-FTA and Brd4-C-HTA targeting were respectively performed on the Brd4 genomic DNA on an androgenetic haploid stem cell line DKO-AG-haESCs. A template for homologous arm amplification was mouse genomic DNA. The correct cell line verified by sequencing was subjected to ICAHCI injection to obtain semi-cloned blastocysts, and the corresponding heterozygous diploid ES cell lines were established (Table 2 and Table 3).

The sequence of Brd4-N-ATF sgRNA (SEQ ID NO:4): TGGGATCACTAGCATGTCTA

The sequence of Brd4-C-FTA sgRNA (SEQ ID NO:5):AATCTTTTTTGAGAGCACCC

The sequence of Brd4-C-HTA sgRNA (SEQ ID NO:6):AATCTTTTTTGAGAGCACCC

The base sequence of Brd4-N-ATF label (SEQ ID NO:7):GGTCTGAACGACATCTTCGAGGCTCAGAAAATCGAATGGCACGAAgagaacctgtacttccagggcGACTACAAAGACCATGACGGTGATTATAAAGATCATGACATCGACT ACAAGGATGACGATGACAAG

The base sequence of Brd4-C-FTA label (SEQ ID NO:8):GACTACAAAGACCATGACGGTGATTATAAAGATCATGACATCGACTACAAGGATGACGATGACAAGgagaacctgtacttccagggcGGTCTGAACGACATCTTCGAGGCTCAGAAAATCGAATGGCACGAA

The base sequence of Brd4-C-HTA label (SEQ ID NO:9):TATCCGTATGATGTGCCGGATTATGCGgagaacctgtacttccagggcGGTCTGAACGACATCTTCGAGGCTCAGAAAATCGAATGGCACGAA

The sequences of the left and right homologous arm amplification primers of Brd4-N-ATF:
Brd4-gN-F4(SEQ ID NO:10):ggctgccatgtagttccagt
Brd4-gN-R4(SEQ ID NO:11):ggcctgcgttgtagacattt
Brd4-gN-F6(SEQ ID NO: 12):ccaagcccagatagatggctagt
Brd4-gN-R2(SEQ ID NO: 13):aaccattcactggggttcagatt

The sequences of the left and right homologous arm amplification primers of Brd4-C-FTA:
Brd4-gC-F(SEQ ID NO:14):gaggagaagattcactcaccaatca
Brd4-gC-R(SEQ ID NO:15):caagccagaatacctagttgcttca

The sequences of the left and right homologous arm amplification primers of Brd4-C- HTA:
Brd4-gC-F(SEQ ID NO:16):gaggagaagattcactcaccaatca
Brd4-gC-R(SEQ ID NO:17):caagccagaatacctagttgcttca

**Table.2 Statistics of Brd4-N-ATF, Brd4-C-FTA and Brd4-C-HTA targeting and establishment of androgenetic haploid cell line**

| | **Exp No.** | **Postive cell lines** | **ICAHCI** | **derived ES cell lines** |
|---|---|---|---|---|
| JJ | Exp098 | Brd4-C-FrA-1/3/4/7/8/11 (6) | Brd4-C-FTA-1/3/4 (3) | |
| JJ | Exp098 | Brd4-C-HTA-3/4/5/7 (4) | Brd4-C-HTA-3/4 (2) | |
| ZL | Exp001 | Brd4-C-HTA-1/2/3/4/5/6 (6) | Brd4-C-HTA-2 (1) | |
| ZF | Exp001 | Brd4-C-FTA-5/6/17/21 (4) | Brd4-C-FTA-5 (1) | |
| ZF | Exp002 | Brd4-N-ATF-2/3/5/6/7/8/9/10 (8) | Brd4-N-ATF-2/3/6/7 (4) | |
| summary | | Brd4-C-FTA (10) | Brd4-C-FTA (4) | Brd4-C-FTA (39) |
| | | Brd4-C-HTA (10) | Brd4-C-HTA (3) | Brd4-C-HTA (22) |
| | | Brd4-N-ATF (8) | Brd4-N-ATF (4) | Brd4-N-ATF (21) |

**Table.3 Statistics of Brd4-N-ATF, Brd4-C-FTA and Brd4-C-HTA diploid ES cell line establishment**

| **Date** | **ICAHCI cell line** | **total** | **2-cell** | **2-cell rate(%)** | **blastocyst** | **blastocyst rate(%)** | **transferred blastocysts** | **derived ES cell lines** | **deriving rate(%)** |
|---|---|---|---|---|---|---|---|---|---|
| 2017-8-1 | Brd4-C-HTA-2 (ZL) | 58 | 55 | 94.8 | 12 | 20.7 | 8 | 3 | 37.5 |
| 2017-8-2 | Brd4-C-FTA-1 (JJ) | 75 | 59 | 78.7 | 25 | 33.3 | 20 | 13 | 65 |
| 2017-8-2 | Brd4-C-FTA-4 (JJ) | 75 | 73 | 97.3 | 29 | 38.7 | 26 | 7 | 26.9 |
| 2017-8-4 | Brd4-C-FTA-3 (JJ) | 48 | 45 | 93.8 | 19 | 39.6 | 17 | 10 | 58.8 |
| 2017-8-4 | Brd4-C-FTA-5 (ZF) | 46 | 41 | 89.1 | 16 | 34.8 | 16 | 9 | 56.3 |
| 2017-8-9 | Brd4-C-HTA-3 (JJ) | 76 | 75 | 98.7 | 41 | 53.9 | 35 | 7 | 20 |
| 2017-8-9 | Brd4-C-HTA-4 (JJ) | 99 | 96 | 96 | 37 | 37.4 | 30 | 12 | 40 |
| 2017-8-10 | Brd4-N-ATF-2 (ZF) | 70 | 66 | 94.3 | 28 | 40 | 19 | 8 | 42.1 |
| 2017-8-10 | Brd4-N-ATF-3 (ZF) | 68 | 62 | 91.2 | 14 | 14.7 | 10 | 5 | 50 |
| 2017-8-11 | Brd4-N-ATF-6 (ZF) | 66 | 64 | 97 | 32 | 48.5 | 26 | 7 | 26.9 |
| 2017-8-11 | Brd4-N-ATF-7 (ZF) | 49 | 40 | 81.6 | 22 | 44.9 | 20 | 1 | 5 |

The TAP-tag-labeled genome copy number was detected by realtime PCR, two pairs of primers were designed for different TAP-tag sequences, and endogenous genomic DNA sequences at the Brd4 N-terminal and C-terminal were used as internal parameters for comparison. Each of the androgenetic haploid embryonic stem cells respectively corresponds to 2 to 4 strains of heterozygous ES cell line (with a symbol "#") established after ICAHCI, and NC represents untargeted androgenetic haploid embryonic stem cells. The results show that the TAP-tag copy number of the androgenetic haploid embryonic stem cells is about 1, and the TAP-tag copy number of the heterozygous ES cell line is about 0.5. It indicates that TAP-tag belongs to site-specific integration and there is no random insertion of a transgene (Table 4).

The sequences of Brd4-N-ATF realtime PCR amplification primers:
FTA-F1(SEQ ID NO: 18):CAAGGATGACGATGACAAGg
FTA-R1(SEQ ID NO: 19):CTGAGCCTCGAAGATGTCGT
FTA-F2(SEQ ID NO:20):CAAGGATGACGATGACAAGg
FTA-R2(SEQ ID NO:21):TTCGTGCCATTCGATTTTCT
ATF-F1(SEQ ID NO:22):CTTCGAGGCTCAGAAAATCG
ATF-R1(SEQ ID NO:23):GTCTTTGTAGTCgccctgga
ATF-F2(SEQ ID NO:24):AAATCGAATGGCACGAAgag
ATF-R2(SEQ ID NO:25):GTCTTTGTAGTCgccctgga
HTA-F2(SEQ ID NO:26):GCGgagaacctgtacttcca
HTA-R2(SEQ ID NO:27):TTCGTGCCATTCGATTTTCT
HTA-F3(SEQ ID NO:28):TATGATGTGCCGGATTATGC
HTA-R3(SEQ ID NO:29):CTGAGCCTCGAAGATGTCGT
Brd4-gN-CN-F(SEQ ID NO:30):gtccacagtggcctttcaat
Brd4-gN-CN-R(SEQ ID NO:31):agctgtcttcagaccctcca
Brd4-gC-CN-F1(SEQ ID NO:32):ttgccttgaacagaccctct
Brd4-gC-CN-R1(SEQ ID NO:33):acacaggtgggaaggaactg
Brd4-gC-CN-F2(SEQ ID NO:34):acagaagcaggagccaaaaa
Brd4-gC-CN-R2(SEQ ID NO:35):aaaggtcaagaggcaggtga

**Table.4 Detection of TAP-tag copy number**

| | **FTA-1/ Brd4-N** | **FTA-1/ Brd4-C-1** | **FTA-1/ Brd4-C-2** | **FTA-2/ Brd4-N** | **FTA-2/ Brd4-C-1** | **FTA-2/ Brd4-C-2** | **AvE ± SD** |
|---|---|---|---|---|---|---|---|
| **NC** | 0.00 | 0.00 | 0.00 | 0.00 | 0.00 | 0.00 | 0.00 ± 0.00 |
| **Brd4-C-FTA-4** | 0.92 | 0.88 | 0.90 | 1.00 | 0.96 | 0.98 | 0.94 ± 0.04 |
| **Brd4-C-FTA-4 5#** | 0.42 | 0.43 | 0.50 | 0.43 | 0.44 | 0.51 | 0.46 ± 0.04 |
| **Brd4-C-FTA-4 6#** | 0.59 | 054 | 062 | 0.71 | 0.66 | 0.76 | 0.65 ± 0.07 |
| **Brd4-C-FTA-5** | 0.77 | 0.72 | 0.87 | 1.01 | 0.95 | 1.15 | 0.91 ± 0.15 |
| **Brd4-C-FTA-5 2#** | 0.68 | 0.58 | 0.73 | 0.73 | 0.63 | 0.79 | 0.69 ± 0.07 |
| **Brd4-CFTA-5 13#** | 0.39 | 039 | 0.47 | 0.30 | 0.30 | 0.37 | 0.37 ± 0.06 |
| | | | | | | | |

| | **ATF-1/ Brd4-N** | **ATF-1/ Brd4-C-1** | **ATF-1/ Brd4-C-2** | **ATF-2/ Brd4-N** | **ATF-2/ Brd4-C-1** | **ATF-2/ Brd4-C-2** | **AVE** ± **SD** |
|---|---|---|---|---|---|---|---|
| **NC** | 0.00 | 0.00 | 0.00 | 0.00 | 0.00 | 0.00 | 0.00 ± 0.00 |
| **Brd4-N-ATF-2** | 1.03 | 1.10 | **1.26** | 0.81 | 0.87 | 1.00 | 1.01 ± 0.15 |
| **Brd4-N-ATF-2 5#** | 0.59 | 0.70 | 0.78 | 0.50 | 0.59 | 0.66 | 0.64 ± 0.09 |
| **Brd4-N-ATF-2 7#** | 0.55 | 0.61 | 0.87 | 0.41 | 0.46 | 0.65 | 0.59 ± 0.15 |
| **Brd4-N-ATF-3** | 0.91 | 0.95 | 1.13 | 0.69 | 0.73 | 0.86 | 0.88 ± 0.14 |
| **Brd4-N-ATF-3 3#** | 0.42 | 0.52 | 0.58 | 0.37 | 0.45 | 0.50 | 0.47 ± 0.07 |
| **Brd4-N-ATF-3 6#** | 0.75 | 0.79 | 0.93 | 0.59 | 0.62 | 0.73 | 0.74 ± 0.11 |
| | | | | | | | |

| | **HTA-2/ Brd4-N** | **HTA-2/ Brd4-C-1** | **HTA-2/ Brd4-C-2** | **HTA-3/ Brd4-N** | **HTA-3/ Brd4-C-1** | **HTA-3/ Brd4-C-2** | **AVE ± SD** |
|---|---|---|---|---|---|---|---|
| **NC** | 0.00 | 0.00 | 0.00 | 0.00 | 0.00 | 0.00 | 0.00 ± 0.00 |
| **Brd4-C-HTA-4** | 0.88 | 1.02 | 1.21 | 0.92 | 1.06 | 1.27 | 1.06 ± 0.14 |
| **Brd4-C-HTA-4 3#** | 0.41 | 0.52 | 0.69 | 0.42 | 0.54 | 0.71 | 0.55 ± 0.12 |
| **Brd4-C-HTA-4 9#** | 0.47 | 0.59 | 0.64 | 0.47 | 0.59 | 0.64 | 0.56 ± 0.07 |
| **Brd4-C-HTA-4 15#** | 0.44 | 0.58 | 0.70 | 0.42 | 0.56 | 0.68 | 0.56 ± 0.11 |
| **Brd4-C-HTA-4 17#** | 0.49 | 0.58 | 0.79 | 0.45 | 0.54 | 0.73 | 0.30 ± 0.12 |

### C. Detection of TAP-tag-labeled Brd4 protein expression level

1 to 2 strains of Brd4-N-ATF, Brd4-C-FTA and Brd4-C-HTA-labeled androgenetic haploid embryonic stem cells (a single number, such as 4) were selected, respectively, corresponded to 2 to 4 strains of ES cell line ("number-number" means, such as 4-5) established after ICAHCI, samples were taken for protein electrophoresis detection, and NC represents untargeted androgenetic haploid embryonic stem cells. By detecting using Flag or HA antibodies, the C-terminal TAP-tag can only specifically detect a Brd4 large protein (about 250 kDa), and the N-terminal can specifically detect Brd4 large protein and small protein (about 120 kDa). However, the protein size is larger than expected. The TAP-tag-labeled Brd4 expression quantity of the heterozygous ES cell line was indeed less than that of the androgenetic haploid embryonic stem cells, but both were expressed. From the point of expression quantity of heterozygous ES cells, the C-terminal TAP-tag was better. A strong extra band (about 150 kDa) was detected by using the Brd4 antibody, and a weak protein signal was detected only near 250 kDa. When the exposure is strong, it can be seen that the band size is changed with the existence of the TAP-tag label, and it indicates that the band is indeed the Brd4 protein. From the results of WB (western blot), both the Flag labeling and HA labeling are successful, the N-terminal and C-terminal TAP-tag labeling of the Brd4 protein are also successful, and the TAP-tag antibody is indeed superior to a Brd4 autoantibody in specificity and sensitivity.

### D. Localization detection of TAP-tag-labeled Brd4 in cells

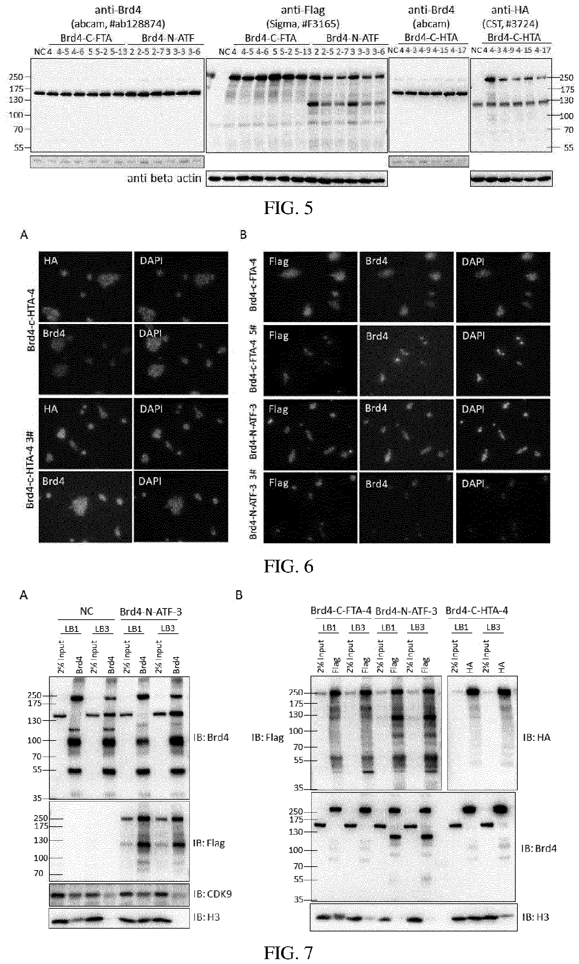

One strain of Brd4-N-ATF, Brd4-C-FTA and Brd4-C-HTA-labeled androgenetic haploid embryonic stem cells and a corresponding ES cell line established after ICAHCI were respectively selected for immunofluorescence assay (IF). Both the HA antibody and the Brd4 antibody were specifically localized in the cell nucleus, and the sensitivity of HA is higher than that of Brd4 by IF assay (Fig. 6A). The Flag antibody can be detected to be localized in the cell nucleus but also localized on the cell membrane (Fig. 6B). It indicates that C-HTA enters the nucleus normally, but some proteins of C-FTA or N-ATF do not enter the nucleus. From the IF results, the TAP-tag of HA is superior to that of Flag.

### E. Co-IP binding protein detection of TAP-tag-labeled Brd4

One strain of Brd4-N-ATF, Brd4-C-FTA and Brd4-C-HTA-labeled androgenetic haploid embryonic stem cells were respectively selected to be subjected to Co-IP binding protein detection of TAP-tag-labeled Brd4. The results show that the NC and Brd4-N-ATF androgenetic haploid embryonic stem cell lines indeed obtain an endogenous Brd4 protein by IP with Brd4 antibody-coupled beads. Both 250 kDa large protein and 110/120 kDa small protein could be detected by the Brd4 antibody. The 150 kDa heteroprotein could also be detected under the LB3 lysate condition. Since the N-terminals of Brd4-N-ATF large and small proteins carried TAP-tag, the molecular weights of the large and small proteins were greater than that of NC. By detecting using the Flag antibody, NC cells were completely negative control, and the Brd4-N-ATF cells could detect the 250 kDa large protein and 120 kDa small protein. Both NC and Brd4-N-ATF cells could be detected binding to the known binding protein CDK9 by Co-IP, but the binding efficiency was lower compared with the input of the total cell lysate before IP, and more proteins were bound under the LB1 lysate condition. The NC and Brd4-N-ATF cells could be detected binding to an H3 protein by Co-IP (Fig. 7A). The Brd4-C-FTA and Brd4-N-ATF androgenetic haploid embryonic stem cell lines could indeed obtain an endogenous Brd4 protein by IP with Flag antibody-coupled beads. By detecting Brd4-C-FTA with Flag and Brd4 antibodies, there was only a 250 kDa large protein, and by detecting Brd4-N-ATF, there were a 250 kDa large protein and a 120 kDa small protein. Since the input of the Brd4 antibody was too high in the expression quantity of the heteroprotein, only the heteroprotein was detected. The Brd4-C-HTA androgenetic haploid embryonic stem cell line could indeed obtain an endogenous Brd4 protein by IP with HA antibody-coupled beads, and only a 250 kDa large protein was detected by the HA and Brd4 antibodies, respectively. From the view of the ratio of input, HA-beads had higher Brd4 binding efficiency compared with Flag-beads, it indicates that HTA tag was better. Both Brd4-C-FTA and Brd4-C-HTA Co-IP could detect binding to H3, and more proteins were bound under the LB1 lysate condition. Brd4-C-HTA bound more than Brd4-C-FTA, it indicates that HTA tag was better. The binding of Brd4-N-ATF to H3 by Co-IP was relatively weak and may be related to the action of small proteins (Fig. 7B). The experiment proves that the Brd4-N-ATF, Brd4-C-FTA and Brd4-C-HTA labeling are correct, the TAP-tag-labeled Brd4 functions normally, it can indeed bind to the reported protein, HTA tag is better than FTA tag, and the LB1 lysate is more suitable for use in co-IP.

### F. Protein expression level detection of other TAP-tag-labeled bromodomain genes

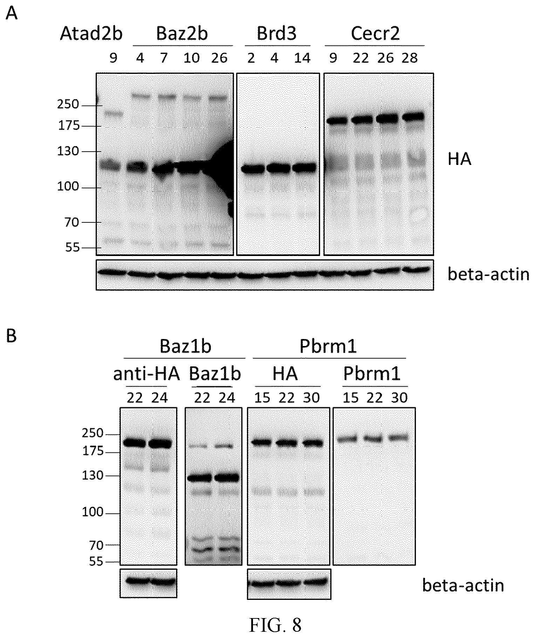

Referring to the above experimental results, Atad2b, Baz2b, Brd3, Cecr2, Bazlb and Pbrm1 in the bromodomain genes were subjected to C-HTA labeling on the androgenetic haploid stem cell line DKO-AG-haESCs, and sgRNA and homologous arms and primers thereof were conventionally designed to detect the protein expression level of the TAP-tag-labeled bromodomain gene. The results show that the cell lines Atad2b-C-HTA-9, Baz2b-C-HTA-4/7/10/26, Brd3-C-HTA-2/4/14, and Cecr2-C-HTA-9/22/26/28 are correctly labeled, expression can be detected with the HA antibody, and the protein size is about 30 kDa larger than expected. Expression intensity: Brd3 (exposing for 5 s) > Cecr2 (30 s) > Atad2b/Baz2b (180 s) (Fig. 8A). The cell lines Bazlb-C-HTA-22/24, and Pbrm1-C-HTA-15/22/30 are both correctly labeled, and expression can be detected with the HA antibody. Expression intensity: Bazlb (HA antibody, exposing for 10 s)> Pbrm1 (HA, 20 s) > Pbrm1 (Pbrm1, 20 s) > Bazlb (Bazlb, 120 s), and the HA antibody is superior to the autoantibody in sensitivity and specificity (Fig. 8B). Moreover, the difference in the expression levels of these TAP-tag labeled proteins can be horizontally compared using only the HA antibody by using the method, thereby realizing the protein expression profile of the whole genomic protein in different tissues.

Related sequences used in the experiments:
sgRNA sequence of Atad2b-C-HTA (SEQ ID NO:36): ACTCAGCATGAGAAGTTCAT
sgRNA sequence of Baz2b-C-HTA (SEQ ID NO:37): ACAACTTCAGCTCACTTTGA
sgRNA sequence of Brd3-C-HTA (SEQ ID NO:38): ACTCAGAGTGAACTCGGACT
sgRNA sequence of Cecr2-C-HTA (SEQ ID NO:39): TGTACTTTCAGAGCTAGTCC
sgRNA sequence of Bazlb-C-HTA (SEQ ID NO:40): CGGAGACAGAAGAAGTAAAG
sgRNA sequence of Pbrml-C-HTA (SEQ ID NO:41): GATGTGATTAAACATTTTCT

The sequences of the left and right homologous arm amplification primers of Atad2b-C-HTA:
Atad2b-gC-F (SEQ ID NO:42): AGGAGCCGCCAGAAATGAAA
Atad2b-gC-R (SEQ ID NO:43): TTTGCCTCTTTGCAACTGCC

The sequences of the left and right homologous arm amplification primers of Baz2b-C-HTA:
Baz2b-gC-F (SEQ ID NO:44): CGGGCGTGACTCGTCTATTA
Baz2b-gC-R (SEQ ID NO:45): TCTATGTGCCTCCAACAGGC

The sequences of the left and right homologous arm amplification primers of Brd3-C-HTA:
Brd3-gC-F (SEQ ID NO:46): CAGATGACAGGTCGTAGCCC
Brd3-gC-R (SEQ ID NO:47): GAACAGGGACCCGTGTCAAA

The sequences of the left and right homologous arm amplification primers of Cecr2-C-HTA:
Cecr2-gC-F (SEQ ID NO:48): AACAGTTGCCACCGCATAAG
Cecr2-gC-R (SEQ ID NO:49): GAGGGAAAACTCCATTGACCCC

The sequences of the left and right homologous arm amplification primers of Bazlb-C-HTA:
Bazlb-gC-F (SEQ ID NO:50): TTGATCGCGGCATCACTTCA
Bazlb-gC-R (SEQ ID NO:51): GATGCTGACACTCCGCTAGA

The sequences of the left and right homologous arm amplification primers of Pbrm1-C-HTA:
Pbrm1-gC-F (SEQ ID NO:52): AGTCTGCCAAGCTGTTCACT
Pbrm1-gC-R (SEQ ID NO:53): ACCACCCAAGCAGGTTCAAA

### Embodiment 2: Construction of Phf7 N-terminal KI Flag tag mouse

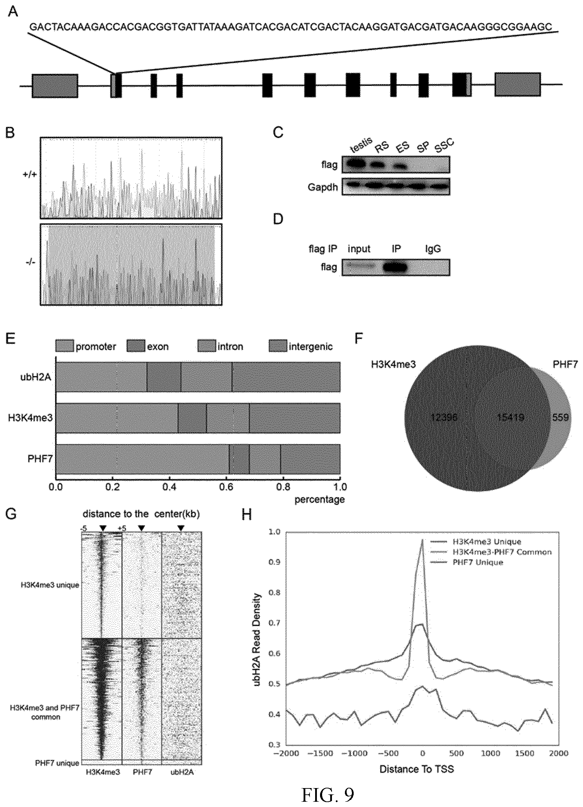

Since there is no good Phf7 antibody on the market, in order to study the function of this gene, a 3×Flag sequence was inserted at the N-terminal of a Phf7 endogenous genome of the androgenetic haploid embryonic stem cell (Fig. 9A), a Phf7-KI-Flag heterozygous mouse F0 was obtained by ICAHCI injection, and a Phf7-KI-Flag homozygous male mouse was obtained by mating between F1 heterozygous mice (Fig. 9B).

The sgRNA sequence of Phf7-N-Flag (SEQ ID NO:54):TTCTAGATAGGAAGGACAGA

The sequences of the left and right homologous arm amplification primers of Phf7-N-Flag:
Phf7-gN-F(SEQ ID NO:55):aaagtagatccccgtggggacac
Phf7-gN-R(SEQ ID NO:56):gtttgtacggctgacaaggagc

The expression of Phf7-Flag was detected in different germ cells isolated from the Phf7-KI-Flag homozygous male mice (Fig. 9C). The expression of Phf7-Flag in the germ cells of the Phf7-KI-Flag homozygous male mice was detected by Co-IP (Fig. 9D). Phf7 was subjected to chip-seq detection by using the Flag antibody and compared with the results of H3K4me3 chip-seq and ubH2A Chip-seq on the exon/intron/intergenic region enrichment situation (Fig. 9E). The Venn diagram shows that peaks of Phf7 chip-seq and H3K4me3 chip-seq binding regions are highly coincident (Fig. 9F). Heatmap shows the signal distribution situation of ubH2A in H3K4me3&Phf7 common, H3K4me3 unique, and Phf7 unique results (Fig. 9G), and specifically counts the signal result value of ubH2A (Fig. 9H). Experiments show that the construction of Phf7-KI-Flag tag mice is free of the restriction of Phf7 antibody, and the functional study of endogenous Phf7 proteins in the tag mice can be completed by using the Flag antibody.

### Embodiment 3: Construction of Hspg2 C-terminal KI Flag mouse

Since there is no good Hspg2 antibody on the market, in order to study the function of this gene, by considering that there is a signal peptide at the N-terminal of Hspg2 protein, a 3 ×Flag sequence was inserted at the C-terminal of an Hspg2 endogenous genome of the androgenetic haploid embryonic stem cell, and an Hspg2-KI-Flag heterozygous mouse was obtained by ICAHCI injection.

The sgRNA sequence of Hspg2-C-Flag (SEQ ID NO: 57): TCATAGGCACCCACCTGCCT

The sequences of the left and right homologous arm amplification primers of Hspg2-C-Flag:
Hspg2-gC-F(SEQ ID NO:58):GTCCTAATGTGGCGGTCAAC
Hspg2-gC-R(SEQ ID NO:59):ACCTCTTCCAGTCCCCTTGTC

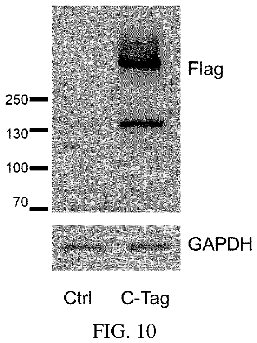

Hspg2-KI-Flag heterozygous mouse embryos at embryonic E15.5 days were taken, and protein electrophoresis was performed on the whole embryo sample to detect the expression of Hspg2-Flag. The result shows that the C-terminal of the Hspg2 protein is successfully labeled (Fig. 10).

The above embodiments are merely illustrative of the principles of the present disclosure and its effects, and are not intended to limit the present disclosure. Any person familiar with the technology may modify or alter the above embodiments without departing from the spirit and scope of the present disclosure. Therefore, all equivalent modifications or alterations made by those with ordinary skill in the art without departing from the spirit and technical idea of the present disclosure should be covered by the appended claims of the present disclosure.

## Claims

1. A high-throughput protein analysis method, comprising: using a tagged semi-cloned mouse library to perform parallel indicator analysis on a plurality of different target proteins of interest with one or several tag protein antibodies; in the tagged semi-cloned mouse library, each semi-cloned mouse is a semi-cloned mouse obtained by culturing after injecting an androgenetic haploid embryonic stem cell into an ovum, or a sexually propagated progeny thereof; the androgenetic haploid embryonic stem cell contains a gene that expresses a fusion protein of a target protein of interest and a tag protein, and the semi-cloned mouse can express the fusion protein of the target protein of interest and the tag protein.

2. The high-throughput protein analysis method according to claim 1, wherein the method further comprises one or more of the following features:
A1) in the fusion protein of the target protein of interest and the tag protein, the tag protein is completely or partially exposed to the surface of the fusion protein;
A2) in the fusion protein of the target protein of interest and the tag protein, the tag protein is located at the N-terminal or C-terminal of the target protein of interest;
A3) the tag protein is selected from one or more of the following: Flag, HA, Green Proteins, Red Proteins, Cyan Proteins, Yellow Proteins, Orange Proteins, Myc, His, GST, Strep, CBP, MBP, iDimerize, ProteoTuner, Shield1, SNAP-tag, CLIP-tag, ACP-tag, MCP-tag, HaloTag, Avi-tag, TAP-tag, Lumio™ tag;
A4) H19 DMR and IG-DMR of the androgenetic haploid embryonic stem cells are knocked out;
A5) the androgenetic haploid embryonic stem cell is from a tagged androgenetic haploid embryonic stem cell library, in the tagged androgenetic haploid embryonic stem cell library, each androgenetic haploid embryonic stem cell contains a gene that expresses a fusion protein of a target protein of interest and a tag protein;
A6) in the tagged semi-cloned mouse library, the tag proteins expressed in fusion with each target protein of interest are the same, or the tag proteins expressed in fusion with each target protein of interest constitute a tag protein combination;
A7) the tagged semi-cloned mouse library is firstly constructed by utilizing a tagged androgenetic haploid embryonic stem cell library, in the tagged androgenetic haploid embryonic stem cell library, each androgenetic haploid embryonic stem cell contains a gene that expresses a fusion protein of a target protein of interest and a tag protein.

3. The high-throughput protein analysis method according to claim 2, wherein in the tagged androgenetic haploid embryonic stem cell library, the tag proteins expressed in fusion with each target protein of interest are the same, or the tag proteins expressed in fusion with each target protein of interest constitute a tag protein combination.

4. The high-throughput protein analysis method according to claim 1, wherein the method is suitable for in vivo, real-time and dynamic analysis.

5. The high-throughput protein analysis method according to claim 1, wherein the protein analysis method does not contain the preparation or use of antibodies of target proteins of interest.

6. A method for constructing the tagged semi-cloned mouse library suitable for the high-throughput protein analysis method described in any one of claims 1-5, comprising the following steps:
1) determining the target protein combination of interest, providing a tagged androgenetic haploid embryonic stem cell library corresponding to the combination, in the tagged androgenetic haploid embryonic stem cell library, each androgenetic haploid embryonic stem cell contains a gene that expresses a fusion protein of a target protein of interest and a tag protein;
2) injecting each androgenetic haploid embryonic stem cell in the tagged androgenetic haploid embryonic stem cell library respectively into an ovum to obtain semi-cloned mice, and screening out the semi-cloned mice that can express the fusion protein of the target protein of interest and the tag protein, the screened primary semi-cloned mice or sexually propagated progeny thereof constitute the tagged semi-cloned mouse library.

7. The method for constructing a tagged semi-cloned mouse library according to claim 6, wherein the tagged semi-cloned mouse library further comprises one or more of the following features:
B1) in the fusion protein of the target protein of interest and the tag protein, the tag protein is completely or partially exposed to the surface of the fusion protein;
B2) in the fusion protein of the target protein of interest and the tag protein, the tag protein is located at the N-terminal or C-terminal of the target protein of interest;
B3) the tag protein is selected from one or more of the following: Flag, HA, Green Proteins, Red Proteins, Cyan Proteins, Yellow Proteins, Orange Proteins, Myc, His, GST, Strep, CBP, MBP, iDimerize, ProteoTuner, Shield1, SNAP-tag, CLIP-tag, ACP-tag, MCP-tag, HaloTag, Avi-tag, TAP-tag, Lumio™ tag;
B4) H19 DMR and IG-DMR of the androgenetic haploid embryonic stem cells are knocked out;
B5) the androgenetic haploid embryonic stem cell is from a tagged androgenetic haploid embryonic stem cell library, in the tagged androgenetic haploid embryonic stem cell library, each androgenetic haploid embryonic stem cell contains a gene that expresses a fusion protein of a target protein of interest and a tag protein;
B6) in the tagged semi-cloned mouse library, the tag proteins expressed in fusion with each target protein of interest are the same, or the tag proteins expressed in fusion with each target protein of interest constitute a tag protein combination.

8. A tagged semi-cloned mouse library suitable for the high-throughput protein analysis method described in any one of claims 1-5, wherein in the tagged semi-cloned mouse library, the target proteins of interest expressed by each semi-cloned mouse are all expressed in fusion with the tag proteins, each semi-cloned mouse is a semi-cloned mouse obtained by culturing after injecting an androgenetic haploid embryonic stem cell into an ovum, or a sexually propagated progeny thereof, and the androgenetic haploid embryonic stem cell contains a gene that expresses a fusion protein of the target protein of interest and the tag protein.

9. The tagged semi-cloned mouse library according to claim 8, wherein the tagged semi-cloned mouse library further comprises one or more of the following features:
C1) in the fusion protein of the target protein of interest and the tag protein, the tag protein is completely or partially exposed to the surface of the fusion protein;
C2) in the fusion protein of the target protein of interest and the tag protein, the tag protein is located at the N-terminal or C-terminal of the target protein of interest;
C3) the tag protein is selected from one or more of the following: Flag, HA, Green Proteins, Red Proteins, Cyan Proteins, Yellow Proteins, Orange Proteins, Myc, His, GST, Strep, CBP, MBP, iDimerize, ProteoTuner, Shield1, SNAP-tag, CLIP-tag, ACP-tag, MCP-tag, HaloTag, Avi-tag, TAP-tag, Lumio™ tag;
C4) H19 DMR and IG-DMR of the androgenetic haploid embryonic stem cells are knocked out;
C5) the androgenetic haploid embryonic stem cell is from a tagged androgenetic haploid embryonic stem cell library;
C6) in the tagged semi-cloned mouse library, the tag proteins expressed in fusion with each target protein of interest are the same, or the tag proteins expressed in fusion with each target protein of interest constitute a tag protein combination;
C7) the tagged semi-cloned mouse library is constructed by the method described in claim 6 or 7.

10. Use of the tagged semi-cloned mouse library according to claim 8 or 9, or semi-cloned mouse from the library, in the fields of protein analysis, protein function research or drug research.

11. A method for constructing a tagged androgenetic haploid embryonic stem cell library suitable for the high-throughput protein analysis method described in any one of claims 1-5, comprising the following steps:
1) determining the target protein combination of interest, performing genetic modification respectively on each androgenetic haploid embryonic stem cell to make them respectively contain a gene that expresses a fusion protein of each target protein of interest and a tag protein in the target protein combination of interest;
2) screening out the androgenetic haploid embryonic stem cell that can express the fusion protein of the target protein of interest and the tag protein;
3) performing reed conservation and library construction on primary cells of the screened androgenetic haploid embryonic stem cells or passage haploid cells thereof to obtain a tagged androgenetic haploid embryonic stem cell library.

12. A method for constructing a tagged androgenetic haploid embryonic stem cell library according to claim 11, wherein the method further comprises one or more of the following features:
D1) in the fusion protein of the target protein of interest and the tag protein, the tag protein is completely or partially exposed to the surface of the fusion protein;
D2) in the fusion protein of the target protein of interest and the tag protein, the tag protein is located at the N-terminal or C-terminal of the target protein of interest;
D3) the tag protein is selected from one or more of the following: Flag, HA, Green Proteins, Red Proteins, Cyan Proteins, Yellow Proteins, Orange Proteins, Myc, His, GST, Strep, CBP, MBP, iDimerize, ProteoTuner, Shield1, SNAP-tag, CLIP-tag, ACP-tag, MCP-tag, HaloTag, Avi-tag, TAP-tag, Lumio™ tag;
D4) H19 DMR and IG-DMR of the androgenetic haploid embryonic stem cells are knocked out;
D5) in the tagged androgenetic haploid embryonic stem cell library, the tag proteins expressed in fusion with each target protein of interest are the same, or the tag proteins expressed in fusion with each target protein of interest constitute a tag protein combination containing a plurality of tag proteins.

13. A tagged androgenetic haploid embryonic stem cell library suitable for the high-throughput protein analysis method described in any one of claims 1-5, wherein in the tagged androgenetic haploid embryonic stem cell library, each androgenetic haploid embryonic stem cell contains a gene that expresses a fusion protein of a target protein of interest and a tag protein, and the semi-cloned mouse obtained by culturing after injecting the androgenetic haploid embryonic stem cell into an ovum can express the fusion protein of the target protein of interest and the tag protein.

14. The tagged androgenetic haploid embryonic stem cell library according to claim 13, wherein the tagged semi-cloned mouse library further comprises one or more of the following features:
in the fusion protein of the target protein of interest and the tag protein, the tag protein is completely or partially exposed to the surface of the fusion protein;
E1) in the fusion protein of the target protein of interest and the tag protein, the tag protein is located at the N-terminal or C-terminal of the target protein of interest;
E2) the tag protein is selected from one or more of the following: Flag, HA, Green Proteins, Red Proteins, Cyan Proteins, Yellow Proteins, Orange Proteins, Myc, His, GST, Strep, CBP, MBP, iDimerize, ProteoTuner, Shield1, SNAP-tag, CLIP-tag, ACP-tag, MCP-tag, HaloTag, Avi-tag, TAP-tag, Lumio™ tag;
E3) H19 DMR and IG-DMR of the androgenetic haploid embryonic stem cells are knocked out;
E4) in the tagged androgenetic haploid embryonic stem cell library, the tag proteins expressed in fusion with each target protein of interest are the same, or the tag proteins expressed in fusion with each target protein of interest constitute a tag protein combination;
E5) the tagged androgenetic haploid embryonic stem cell library is constructed according to the method described in claim 10 or 11.

15. Use of the tagged androgenetic haploid embryonic stem cell library according to claim 13 or 14, or androgenetic haploid embryonic stem cells from the library, in the fields of protein analysis, protein function research or drug research.
